(19) Europäisches Patentamt / European Patent Office / Office européen des brevets

(11) **EP 4 369 357 A1**

(12) **EUROPEAN PATENT APPLICATION**
published in accordance with Art. 153(4) EPC

(43) Date of publication:
**15.05.2024 Bulletin 2024/20**

(21) Application number: **22860383.3**

(22) Date of filing: **18.08.2022**

(51) International Patent Classification (IPC):
**G16H 50/30** (2018.01)

(52) Cooperative Patent Classification (CPC):
**G16H 10/60; G16H 20/30; G16H 50/30**

(86) International application number:
**PCT/CN2022/113387**

(87) International publication number:
**WO 2023/025037 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.08.2021 CN 202111000243**

(71) Applicant: **Huawei Technologies Co., Ltd.
Shenzhen, Guangdong 518129 (CN)**

(72) Inventors:
• **YANG, Jian
Shenzhen, Guangdong 518129 (CN)**
• **DONG, Wei
Shenzhen, Guangdong 518129 (CN)**
• **LI, Yang
Shenzhen, Guangdong 518129 (CN)**
• **SU, Cheng
Shenzhen, Guangdong 518129 (CN)**
• **LI, Xuechen
Shenzhen, Guangdong 518129 (CN)**
• **YU, Ye
Shenzhen, Guangdong 518129 (CN)**
• **ZHU, Zhou
Shenzhen, Guangdong 518129 (CN)**

(74) Representative: **Epping - Hermann - Fischer
Patentanwaltsgesellschaft mbH
Schloßschmidstraße 5
80639 München (DE)**

(54) **HEALTH MANAGEMENT METHOD AND SYSTEM, AND ELECTRONIC DEVICE**

(57) A health management method, a system, and an electronic device are provided. In this method, user data is used to help a user assess health risk factors currently exposed, assess overall health risk factors of the user, provide a personalized comprehensive intervention plan for controllable risk factors closely related to individual health, and predict health benefits of the intervention plan. After a phase of the intervention plan is implemented, an intervention effect assessment result may be further provided, and a next phase of the intervention plan can be adjusted, to promote development of a healthy life of the user and achievement of an active health management objective.

FIG. 4A

EP 4 369 357 A1

CONT.
FROM
FIG. 4A

CONT.
FROM
FIG. 4A

CONT.
FROM
FIG. 4A

(5)
Data
monitoring

S406: Monitor
actual execution
data of the
intervention plan
and health data

S407:
Measure the
health data

S408: Monitor the
actual execution
data of the
intervention plan

S409: Monitor the
actual execution
data of the
intervention plan
and the health data

S410: Send the
actual execution data
and the health data

(6)
Effect
assessment
and
intervention
plan
adjustment

S411: Run an effect assessment
model to assess an effect of an
actual execution status of an
intervention plan of a current
cycle, and provide a
recommendation

S412: Adjust an intervention
plan of a next cycle based on
an assessment result

FIG. 4B

## Description

This application claims priority to Chinese Patent Application No. 202111000243.1, filed with the China National Intellectual Property Administration on August 27, 2021 and entitled "HEALTH MANAGEMENT METHOD, SYSTEM, AND ELECTRONIC DEVICE", which is incorporated herein by reference in its entirety.

## TECHNICAL FIELD

This application relates to the field of terminals and communication technologies, and in particular, to a health management method, a system, and an electronic device.

## BACKGROUND

With the continuous improvement of living standards of residents, unhealthy lifestyles lead to a rising prevalence rate of chronic non-communicable diseases (obesity, cardiovascular disease, diabetes, cancer, chronic obstructive pulmonary disease, and the like) in our country and a younger age of onset. This places a serious burden on families and society. The occurrence and development of disease is a long-term evolution and change process, and is a result of a combination of genetic, physiological, environmental, and behavioral factors.

A user lacks recognition and intervention management on controllable risk factors (insufficient physical activity, unhealthy diet, stress, sleep deprivation, and the like) of the user in a daily life. In addition, even if the user knows a close relationship between bad lifestyles and health of the user, before a disease is diagnosed or seriously affects quality of life, it is difficult for the user to improve lifestyles and keep good lifestyles, and the user has low compliance with good habits and behaviors. Therefore, how to promote the user to perform active health management becomes a problem to be urgently resolved.

## SUMMARY

This application provides a health management method, a system, and an electronic device, to perform health management of a user, and promote achievement of an active health management objective of the user.

According to a first aspect, this application provides a health management method. The method includes: An electronic device obtains a first intervention plan generated based on user data of a first user. The electronic device predicts a predicted value of a health indicator obtained after a part and/or all of the first intervention plan is completed.

The first intervention plan may include a first exercise plan, and/or a first diet plan, and/or a first health habit check-in task set.

In the foregoing implementation, the electronic device may predict a predicted value of a health indicator obtained after a part and/or all of an intervention plan of a user is completed. The predicted value can greatly improve confidence and subjective initiative of the user in executing the intervention plan, and promotes achievement of an active health management objective of the user.

With reference to some implementations of the first aspect, in some implementations, that an electronic device obtains a first intervention plan generated based on user data of a first user specifically includes: The electronic device obtains the user data of the first user. The electronic device generates the first intervention plan based on the user data.

In the foregoing implementations, the electronic device generates the first intervention plan based on the obtained user data of the first user. This improves effectiveness of the generated first intervention plan, and promotes the achievement of the active health management objective of the user.

With reference to some implementations of the first aspect, in some implementations, that the electronic device generates the first intervention plan based on the user data specifically includes: The electronic device recognizes a user health risk factor based on the user data. The electronic device generates the first intervention plan for the user health risk factor.

In the foregoing implementations, the electronic device may first recognize the user health risk factor of the first user based on the user data of the first user, and then generate the first intervention plan for the user health risk factor. In this way, the generated intervention plan is more targeted to the first user. This improves effectiveness of the generated intervention plan for user health management of the first user.

With reference to some implementations of the first aspect, in some implementations, the user data includes basic user information, user behavior data, and/or user health data.

In the foregoing implementations, the user data may include not only the basic user information, but also the user behavior data and/or the user health data, so that the electronic device can recognize the user health risk factor of the first user based on personalized information of the first user, and formulate the corresponding intervention plan. This promotes the achievement of the active health management objective of the user.

With reference to some implementations of the first aspect, in some implementations, the basic user information includes an age and/or a gender; the user behavior data includes at least one of exercise data, stress data, sleep data, diet data, drinking data, and smoking data; and the user health data includes at least one of body weight data, body composition data, blood pressure data, blood glucose data, and blood lipid data.

In the foregoing implementations, the user data includes data related to various aspects such as a personal behavior and a health characteristic of the user. This improves accuracy of the recognized user health risk factor, and also improves effectiveness of the formulated first intervention plan. With reference to some implementations of the first aspect, in some implementations, that the electronic device recognizes a user health risk factor based on the user data specifically includes: The electronic device obtains, from risk factor correspondences of a plurality of groups, a risk factor correspondence of a first group corresponding to the basic user information, where risk factor correspondences of different groups correspond to different age ranges and/or genders, and the risk factor correspondence of the first group includes a correspondence between one or more health risk factors and a corresponding preset condition of the one or more health risk factors, and includes a correspondence between a first health risk factor and a first preset condition. The electronic device determines the user health risk factor based on the user behavior data and/or the user health data and with reference to the risk factor correspondence of the first group. When the user behavior data and/or the user health data meet/ meets the first preset condition, the user health risk factor includes the first health risk factor.

In the foregoing implementations, a risk factor correspondence of one group includes a correspondence between one or more health risk factors and a corresponding preset condition of the one or more health risk factors. For example, the risk factor correspondence may include the correspondence between the first health risk factor and the first preset condition, and may further include a correspondence between a second health risk factor and a second preset condition. When the user behavior data and/or the user health data of the first user meet/meets the first preset condition, the electronic device may determine that the first health risk factor is a health risk factor in the user health risk factor of the first user. Similarly, when the user behavior data and/or the user health data of the first user meet/meets the second preset condition, the electronic device may determine that the second health risk factor is another health risk factor in the user health risk factor of the first user. It may be understood that, if the user behavior data and/or the user health data of the first user do/does not meet the second preset condition, the electronic device may determine that the second health risk factor is not a health risk factor in the user health risk factor of the first user. In this manner, the user health risk factor of the first user can be effectively, accurately, and more comprehensively recognized, to provide a favorable condition for accurately performing health management on the first user.

With reference to some implementations of the first aspect, in some implementations, for any health risk factor in the risk factor correspondence, a preset condition corresponding to the health risk factor may include a more refined preset subcondition, to present a risk degree of a health risk factor corresponding to the preset subcondition. That the electronic device determines the user health risk factor based on the user behavior data and/or the user health data and with reference to the risk factor correspondence of the first group may specifically include: The electronic device determines the user health risk factor and an exposure level of one or more health risk factors of the user health risk factor based on the user behavior data and/or the user health data and with reference to the risk factor correspondence of the first group. The electronic device generates the first intervention plan for the user health risk factor and the exposure level of the one or more health risk factors.

In the foregoing implementations, not only the user health risk factor can be determined, but also the exposure level of the one or more health risk factors can be determined, to more accurately determine which health risk factors have the greatest impact on current health of the user. An exposure level of the health risk factor is also used as a reference for generation of an intervention plan. In this way, a more targeted intervention plan for a health risk factor with a higher risk level can be generated, to improve an intervention effect of the generated first intervention plan.

With reference to some implementations of the first aspect, in some implementations, that the electronic device predicts a predicted value of a health indicator obtained after a part and/or all of the first intervention plan is completed specifically includes: The electronic device performs model training based on individual training data of the first user, to obtain a first individual health benefit prediction model. The individual training data of the first user includes an execution status of a historical intervention plan of the first user, a value of the health indicator before the historical intervention plan of the first user is executed, and a value of the health indicator after a part and/or all of the historical intervention plan of the first user is executed. The electronic device inputs the user health data in the user data and the part and/or all of the first intervention plan into the first individual health benefit prediction model, to predict the predicted value of the health indicator obtained after the part and/or all of the first intervention plan is completed.

In the foregoing implementations, the first individual health benefit prediction model is obtained through training by using a machine learning algorithm, and then the model is used to predict the predicted value of the health indicator obtained after the part and/or all of the first intervention plan is completed. No human intervention is required in the prediction process. This improves efficiency of obtaining the predicted value of the health indicator, and also improves accuracy of the obtained predicted value of the health indicator.

Optionally, the electronic device may perform model training by using the machine learning algorithm to obtain the first individual health benefit prediction model in a plurality of manners.

For example, the electronic device may first obtain a basic group model of the first group corresponding to the basic user information in the user data. The basic group model of the first group is one of basic group models of a plurality of groups. Different groups correspond to different age ranges and/or genders. For any basic group model in the basic group models of the plurality of groups, the basic group model is obtained by training by using the machine learning algorithm based on group training data of a group corresponding to the basic group model. The group training data of the corresponding group includes an execution status of a user intervention plan of the group corresponding to the basic group model, a value of the health indicator before the intervention plan is executed, and a value of the health indicator after a part and/or all of the intervention plan is executed.

Then, the electronic device may train and optimize the basic group model of the first group based on the individual training data of the first user, to obtain the first individual health benefit prediction model.

In the foregoing implementations, the first individual health benefit prediction model dedicated to the first user is obtained based on the basic group model and with reference to the individual training data of the first user. The first individual health benefit prediction model is used to predict the predicted value of the health indicator obtained after the part and/or all of the first intervention plan of the first user is completed. In this way, privacy of training data for training a health benefit prediction model can be ensured, and prediction accuracy of the health benefit prediction model can be improved.

For another example, the electronic device may directly use the individual training data of the first user to perform model training by using the machine learning algorithm, to obtain the first individual health benefit prediction model. This is not limited herein.

With reference to some implementations of the first aspect, in some implementations, the health indicator includes at least one of a body weight, a body mass index, a body fat percentage, systolic blood pressure, diastolic blood pressure, fasting plasma glucose, total cholesterol, and triglyceride. In the foregoing implementations, the health indicator may include a plurality of indicators related to user health, to improve comprehensiveness and effectiveness of user health management. With reference to some implementations of the first aspect, in some implementations, the method further includes: The electronic device displays the predicted value of the health indicator obtained after the part and/or all of the first intervention plan is completed.

In the foregoing implementations, the predicted value of the health indicator obtained after the part and/or all of the first intervention plan is completed is displayed to the user. This improves confidence and subjective initiative of the user in executing the intervention plan, and promotes the achievement of the active health management objective of the user.

With reference to some implementations of the first aspect, in some implementations, the first intervention plan includes intervention plans of N cycles, N is a positive integer greater than 1, and the predicted value of the health indicator obtained after the part and/or all of the first intervention plan is completed includes predicted values of the health indicator obtained after a part of and/or all cycles in the intervention plans of N cycles in the first intervention plan are separately completed. That the electronic device displays the predicted value of the health indicator obtained after the part and/or all of the first intervention plan is completed specifically includes: The electronic device displays a change trend of the health indicator obtained after the first intervention plan is completed. The change trend of the health indicator includes the predicted values of the health indicator obtained after the part of and/or all cycles in the intervention plans of N cycles in the first intervention plan are separately completed.

In the foregoing implementations, the first intervention plan includes intervention plans of a plurality of cycles. The electronic device may predict predicted values of the health indicator obtained after the intervention plans of the plurality of cycles in the first intervention plan are separately completed, and display the predicted values of the health indicator to the user in a change trend manner. This can improve confidence and subjective initiative of the user in executing the intervention plan, and promotes the achievement of the active health management objective of the user.

With reference to some implementations of the first aspect, in some implementations, a health management system in which the electronic device is located further includes an intelligent wearable device, and/or a health check device, and/or an intelligent fitness device. The method further includes: The electronic device delivers a wearable intervention sub-plan in the first intervention plan to the intelligent wearable device. The wearable intervention sub-plan is a part or all of the first intervention plan. The electronic device delivers a check intervention sub-plan in the first intervention plan to the health check device. The check intervention sub-plan is a part or all of the first intervention plan. The electronic device delivers a fitness intervention sub-plan in the first intervention plan to the intelligent fitness device. The fitness intervention sub-plan is a part or all of the first intervention plan. The wearable intervention sub-plan, the check intervention sub-plan, and the fitness intervention sub-plan are the same or different. The wearable intervention sub-plan is a plan that is in the first intervention plan and that is to be executed by the intelligent wearable device. The check intervention sub-plan is a plan that is in the first intervention plan and that is to be executed by the health check device. The fitness intervention sub-plan is a plan that is in the first intervention plan and that is to be executed by the intelligent fitness device.

In the foregoing implementations, the electronic device may separately deliver a part or all of intervention plans of various types in the first intervention plan to another corresponding device in the health management system, for example, deliver the wearable intervention sub-plan in the first intervention plan to the intelligent wearable device, deliver the check intervention sub-plan in the first intervention plan to the health check device, and deliver the fitness intervention sub-plan in the first intervention plan to the intelligent fitness device, so that devices in the health management system can coordinate to promote the achievement of the active health management objective of the user.

With reference to some implementations of the first aspect, in some implementations, the first intervention plan includes a first exercise plan, and/or a first diet plan, and/or a first health habit check-in task set. The wearable intervention sub-plan includes a part or all of the first exercise plan, and/or a part or all of the first diet plan, and/or a part or all of the first health habit check-in task set. The check intervention sub-plan includes a part or all of health indicator check tasks in the first health habit check-in task set. The fitness intervention sub-plan includes a part or all of the first exercise plan.

In the foregoing implementations, the first intervention plan may include plans related to a plurality of aspects of health of the user, for example, an exercise plan, a diet plan, and a health habit check-in task set. These plans of a plurality of aspects may be separately partially or completely included in an intervention plan delivered to another device in the health management system. This improves effectiveness that the devices in the health management system can coordinate to promote the achievement of the active health management objective of the user.

With reference to some implementations of the first aspect, in some implementations, the first intervention plan includes the intervention plans of N cycles, and N is a positive integer greater than 1. The wearable intervention sub-plan is a wearable intervention sub-plan of one or all cycles in the first intervention plan. The check intervention sub-plan is a check intervention sub-plan of one or all cycles in the first intervention plan. The fitness intervention sub-plan is a fitness intervention sub-plan of one or all cycles in the first intervention plan.

In the foregoing implementations, the intervention plan delivered to the another device in the health management system may be an intervention plan of one cycle. In this way, an amount of data sent by the electronic device can be reduced, and power consumption of the electronic device can be reduced. Alternatively, an intervention plan of all cycles may be delivered. In this way, when a network between the electronic device and the another device is disconnected, the another device can also complete the intervention plan of all cycles. This improves anti-interference performance of the health management system.

With reference to some implementations of the first aspect, in some implementations, the first intervention plan includes the intervention plans of N cycles, and N is a positive integer greater than 1. The method further includes: The electronic device obtains actual execution data and/or a value of the health indicator in an execution process of a first cycle in the first intervention plan. The actual execution data and/or the value of the health indicator are/is obtained through monitoring by the electronic device and/or the another device in the health management system in which the electronic device is located.

In the foregoing implementations, the electronic device may obtain, through monitoring by the electronic device and/or the another device in the health management system, the actual execution data and/or the value of the health indicator in the execution process of the first cycle in the first intervention plan, to provide data support for accurately performing user health management.

It may be understood that, the actual execution data and/or the value of the health indicator in the execution process of the first cycle in the first intervention plan include/includes a value of the health indicator obtained after execution of the first cycle in the first intervention plan is completed. With reference to some implementations of the first aspect, in some implementations, the another device in the health management system in which the electronic device is located includes the intelligent wearable device, and/or the health check device, and/or the intelligent fitness device. That the electronic device obtains actual execution data and/or a value of the health indicator in an execution process of a first cycle in the first intervention plan specifically includes: The electronic device monitors first-part actual execution data and/or values of first-part health indicators in the execution process of the first cycle in the first intervention plan on the electronic device. The electronic device receives second-part actual execution data that is monitored by the intelligent fitness device and that is in the execution process of the first cycle in the first intervention plan. The electronic device receives values of second-part health indicators that are measured by the health check device and that are in the execution process of the first cycle in the first intervention plan. The electronic device receives third-part actual execution data and/or values of third-part health indicators that are/is monitored by the intelligent wearable device and that are/is in the execution process of the first cycle in the first intervention plan. The electronic device uses the first-part actual execution data, the second-part actual execution data, and the third-part actual execution data as the actual execution data in the execution process of the first cycle, and uses the values of the first-part health indicators, the values of the second-part health indicators, and the values of the third-part health indicators as the value of the health indicator in the execution process of the first cycle.

In the foregoing implementations, the another device in the health management system may include the intelligent wearable device, and/or the health check device, and/or the intelligent fitness device. The devices in the health management system jointly promote the user to complete an intervention plan, to effectively improve a possibility that the user achieves the active health management objective.

With reference to some implementations of the first aspect, in some implementations, the predicted value of the health indicator obtained after the part and/or all of the first intervention plan is completed includes a predicted value of the health indicator obtained after the first cycle in the first intervention plan is completed. The method further includes: The electronic device compares the predicted value of the health indicator obtained after the first cycle in the first intervention plan is completed and an actual value of the health indicator obtained after an intervention plan of the first cycle is completed for a degree of consistency between the values, to obtain an intervention effect assessment result.

In the foregoing implementations, after the first intervention plan is completed, the predicted value of the health indicator and the actual value of the health indicator obtained after the intervention plan of the first cycle is completed may be compared for the degree of consistency between the values, to obtain the intervention effect assessment result, so that an effect of the first intervention plan can be more accurately assessed.

With reference to some implementations of the first aspect, in some implementations, the method further includes: The electronic device generates an assessment of one or more plans in the first intervention plan based on the intervention effect assessment result and with reference to the actual execution data and/or the value of the health indicator in the execution process of the first cycle, and uses the assessment as an assessment result of the first intervention plan.

In the foregoing implementations, the assessment result of the first intervention plan may be generated with reference to the actual execution data and/or the value of the health indicator, so that whether the first intervention plan is suitable for the first user can be more accurately assessed. With reference to some implementations of the first aspect, in some implementations, the method further includes: The electronic device adjusts an intervention plan of a second cycle in the first intervention plan based on the assessment result of the first intervention plan. The second cycle is a next cycle of the first cycle.

In the foregoing implementations, an intervention plan of a next cycle in the first intervention plan may be adjusted based on the assessment result of the first intervention plan, to implement closed-loop health management, improve user experience, and achieve a long-term health promotion effect.

With reference to some implementations of the first aspect, in some implementations, after adjusting the intervention plan of the second cycle in the first intervention plan, the method further includes: The electronic device predicts a predicted value of the health indicator obtained after a part and/or all of an adjusted first intervention plan is completed.

With reference to some implementations of the first aspect, in some implementations, the method further includes: The electronic device displays a change trend of the health indicator obtained after the part and/or all of the adjusted first intervention plan is completed.

In the foregoing implementations, the electronic device may predict the predicted value of the health indicator obtained after the part and/or all of the adjusted first intervention plan is completed, and display the predicted value to the user. This can improve confidence and subjective initiative of the user in executing the adjusted first intervention plan, and promote achievement of the active health management objective of the user.

With reference to some implementations of the first aspect, in some implementations, the method further includes: The electronic device delivers the part and/or all of the adjusted first intervention plan to the another device in the health management system in which the electronic device is located.

In the foregoing implementations, after adjusting the first intervention plan, the electronic device may deliver the part and/or all of the adjusted first intervention plan to the another device in the health management system in which the electronic device is located, to update the first intervention plan stored in the another device. In this way, each device in the health management system can promote the achievement of the active health management objective of the user according to the latest first intervention plan, to improve a health management effect.

With reference to some implementations of the first aspect, in some implementations, the method further includes: The electronic device estimates the age of the first user based on the user data, and uses the age as an estimated user age of the first user. The user data includes the user behavior data and/or the user health data. The electronic device predicts a predicted value of an estimated user age of the first user obtained after the part and/or all of the first intervention plan is completed. In the foregoing implementations, the electronic device may obtain the estimated user age of the first user through estimation based on the user data, and can predict the predicted value of the estimated user age of the first user obtained after the user completes the part and/or all of the first intervention plan. This can concisely reflect an overall health risk status of the user.

With reference to some implementations of the first aspect, in some implementations, the method further includes: The electronic device displays the estimated user age and the predicted value of the estimated user age.

In the foregoing implementations, the electronic device may display the estimated user age and the predicted value of the estimated user age to the user, to improve subjective initiative of the user in executing the first intervention plan, and promote the achievement of the active health management objective of the user.

With reference to some implementations of the first aspect, in some implementations, the method further includes: determining a baseline mortality rate for each cause-of-death disease of a first group corresponding to user basic information in the user data in a risk-free exposure case, where the risk-free exposure case is a hypothetical case in which everyone in the group has no health risk factor; determining group life expectancy of the first group; determining user life expectancy based on the user health risk factor and the baseline mortality rate for each cause-of-death disease of the first group in the risk-free exposure case; and determining the estimated user age of the first user based on the user life expectancy, the group life expectancy of the first group, and an actual age of the first user.

In the foregoing implementations, in a process of determining the estimated user age, not only the user health risk factor and the actual age that are specific to the first user are referred to, but also the group life expectancy of the first group to which the first user belongs is referred to, so that the estimated user age of the first user obtained through estimation can more accurately reflect an overall health status of the user.

According to a second aspect, this application provides an intervention plan assessment method. The method includes: An electronic device obtains a predicted value of a health indicator obtained after a part and/or all of a first intervention plan is completed. The electronic device obtains actual execution data and/or a value of the health indicator in an execution process of the first intervention plan. The electronic device compares the predicted value of the health indicator obtained after the part and/or all of the first intervention plan is completed and an actual value of the health indicator obtained after the part and/or all of the first intervention plan is completed for a degree of consistency between the values, to obtain an intervention effect assessment result.

In the foregoing implementations, after the first intervention plan is completed, the predicted value of the health indicator and the actual value of the health indicator obtained after the intervention plan of the first cycle is completed may be compared for the degree of consistency between the values, to obtain the intervention effect assessment result, so that an effect of the first intervention plan can be more accurately assessed.

With reference to some implementations of the second aspect, in some implementations, the first intervention plan includes intervention plans of N cycles, N is a positive integer greater than 1, and the predicted value of the health indicator obtained after the part and/or all of the first intervention plan is completed includes a predicted value of the health indicator obtained after a first cycle in the first intervention plan is completed. That the electronic device obtains actual execution data and/or a value of the health indicator in an execution process of the first intervention plan specifically includes: The electronic device obtains actual execution data and/or a value of the health indicator in an execution process of the first cycle in the first intervention plan. The actual execution data and/or the value of the health indicator are/is obtained through monitoring by the electronic device and/or another device in a health management system in which the electronic device is located. That the electronic device compares the predicted value of the health indicator obtained after the part and/or all of the first intervention plan is completed and an actual value of the health indicator obtained after the part and/or all of the first intervention plan is completed for a degree of consistency between the values, to obtain an intervention effect assessment result specifically includes: The electronic device compares the predicted value of the health indicator obtained after the first cycle in the first intervention plan is completed and an actual value of the health indicator obtained after an intervention plan of the first cycle is completed for a degree of consistency between the values, to obtain the intervention effect assessment result.

With reference to some implementations of the second aspect, in some implementations, the another device in the health management system in which the electronic device is located includes an intelligent wearable device, and/or a health check device, and/or an intelligent fitness device.

In the foregoing implementations, the another device in the health management system may include the intelligent wearable device, and/or the health check device, and/or the intelligent fitness device. The devices in the health management system jointly promote the user to complete an intervention plan, to effectively improve a possibility that the user achieves the active health management objective.

With reference to some implementations of the second aspect, in some implementations, the method further includes: The electronic device generates an assessment of one or more plans in the first intervention plan based on the intervention effect assessment result and with reference to the actual execution data and/or the value of the health indicator in the execution process of the first cycle, and uses the assessment as an assessment result of the first intervention plan.

In the foregoing implementations, the assessment result of the first intervention plan may be generated with reference to the actual execution data and/or the value of the health indicator, so that whether the first intervention plan is suitable for a first user can be more accurately assessed. With reference to some implementations of the second aspect, in some implementations, the method further includes: The electronic device adjusts an intervention plan of a second cycle in the first intervention plan based on the assessment result of the first intervention plan. The second cycle is a next cycle of the first cycle.

In the foregoing implementations, an intervention plan of a next cycle in the first intervention plan may be adjusted based on the assessment result of the first intervention plan, to implement closed-loop health management, improve user experience, and achieve a long-term health promotion effect.

With reference to some implementations of the second aspect, in some implementations, that the electronic device compares the predicted value of the health indicator obtained after the first cycle in the first intervention plan is completed and an actual value of the health indicator obtained after an intervention plan of the first cycle is completed for a degree of consistency between the values, to obtain the intervention effect assessment result specifically includes: The electronic device compares the predicted value of the health indicator obtained after the first cycle in the first intervention plan is completed and the actual value of the health indicator obtained after the intervention plan of the first cycle is completed for the degree of consistency between the values, and compares a predicted value of an estimated user age obtained after the first cycle in the first intervention plan is completed and an estimated user age obtained after the intervention plan of the first cycle is actually completed for a degree of consistency between the values, to obtain the intervention effect assessment result.

In the foregoing implementations, an estimated user age may be added in an intervention effect assessment process. Because the estimated user age can reflect an overall health status of the user, the intervention effect assessment result can be more accurate.

With reference to some implementations of the second aspect, in some implementations, the method further includes: The electronic device obtains the first intervention plan generated based on user data of the first user. That an electronic device obtains a predicted value of a health indicator obtained after a part and/or all of a first intervention plan is completed specifically includes: The electronic device predicts the predicted value of the health indicator obtained after the part and/or all of the first intervention plan is completed.

In the foregoing implementations, the electronic device may generate the first intervention plan and predict the predicted value of the health indicator obtained after the part and/or all of the intervention plan is completed. This improves confidence and subjective initiative of the user in executing the intervention plan, and promotes the achievement of the active health management objective of the user.

According to a third aspect, this application provides a user age estimation method, including: An electronic device obtains user data of a first user. The user data includes user behavior data and/or user health data. The electronic device estimates an age of the first user based on the user data of the first user, and uses the age as an estimated user age of the first user.

In the foregoing implementation, the electronic device may obtain the estimated user age of the first user through estimation based on the user data. This can concisely reflect an overall health risk status of the user.

With reference to some implementations of the third aspect, in some implementations, that the electronic device estimates an age of the first user based on the user data of the first user, and uses the age as an estimated user age of the first user specifically includes: The electronic device recognizes a user health risk factor based on the user data. The electronic device estimates the age of the first user based on the user data and the user health risk factor, and uses the age as the estimated user age of the first user.

In the foregoing implementations, in a process of estimating the estimated user age of the first user, the electronic device first needs to recognize the user health risk factor, so that the obtained estimated user age can more accurately reflect an overall health level of the user.

With reference to some implementations of the third aspect, in some implementations, the method further includes: The electronic device generates a first intervention plan for the user health risk factor. The electronic device predicts a predicted value of an estimated user age of the first user obtained after a part and/or all of the first intervention plan is completed.

In the foregoing implementations, the electronic device may predict the estimated user age of the user obtained after the first intervention plan is completed. This improves subjective initiative of the user in executing the intervention plan, and promotes achievement of an active health management objective of the user.

With reference to some implementations of the third aspect, in some implementations, that the electronic device estimates the age of the first user based on the user data and the user health risk factor, and uses the age as the estimated user age of the first user specifically includes: determining a baseline mortality rate for each cause-of-death disease of a first group corresponding to user basic information in the user data in a risk-free exposure case, where the risk-free exposure case is a hypothetical case in which everyone in the group has no health risk factor; determining group life expectancy of the first group; determining user life expectancy based on the user health risk factor and the baseline mortality rate for each cause-of-death disease of the first group in the risk-free exposure case; and determining the estimated user age of the first user based on the user life expectancy, the group life expectancy of the first group, and an actual age of the first user.

In the foregoing implementations, in a process of determining the estimated user age, not only the user health risk factor and the actual age that are specific to the first user are referred to, but also the group life expectancy of the first group to which the first user belongs is referred to, so that the estimated user age of the first user obtained through estimation can more accurately reflect an overall health status of the user.

According to a fourth aspect, an embodiment of this application provides an electronic device. The electronic device includes one or more processors and a memory, the memory is coupled to the one or more processors, the memory is configured to store computer program code, the computer program code includes computer instructions, and the one or more processors invoke the computer instructions, so that the electronic device performs the method described in the first aspect or any possible implementation of the first aspect, the second aspect or any possible implementation of the second aspect, and the third aspect or any possible implementation of the third aspect. According to a fifth aspect, this application provides a chip system. The chip system is used in an electronic device, the chip system includes one or more processors, and the processor is configured to invoke computer instructions, so that the electronic device performs the method described in the first aspect or any possible implementation of the first aspect, the second aspect or any possible implementation of the second aspect, and the third aspect or any possible implementation of the third aspect.

According to a sixth aspect, this application provides a computer program product including instructions. When the computer program product runs on an electronic device, the electronic device is enabled to perform the method described in the first aspect or any possible implementation of the first aspect, the second aspect or any possible implementation of the second aspect, and the third aspect or any possible implementation of the third aspect.

According to a seventh aspect, this application provides a computer-readable storage medium, including instructions. When the instructions are run on an electronic device, the electronic device is enabled to perform the method described in the first aspect or any possible implementation of the first aspect, the second aspect or any possible implementation of the second aspect, and the third aspect or any possible implementation of the third aspect.

According to an eighth aspect, this application provides a user health management system. The health management system includes an electronic device and at least one of an intelligent wearable device, a health check device, or an intelligent fitness device. The electronic device is configured to perform the method described in the first aspect or any possible implementation of the first aspect, the second aspect or any possible implementation of the second aspect, and the third aspect or any possible implementation of the third aspect.

It may be understood that, the electronic device provided in the fourth aspect, the chip system provided in the fifth aspect, the computer program product provided in the sixth aspect, the computer storage medium provided in the seventh aspect, and the user health management system provided in the eighth aspect are all configured to perform the method provided in the first aspect or any possible implementation of the first aspect, the second aspect or any possible implementation of the second aspect, and the third aspect or any possible implementation of the third aspect of this application. Therefore, for beneficial effects that can be achieved by the electronic device, the chip system, the computer program product, the computer storage medium, and the user health management system, refer to beneficial effects in the corresponding method. Details are not described herein again.

## BRIEF DESCRIPTION OF DRAWINGS

FIG. 1 shows a disease risk assessment manner according to an embodiment of this application;
FIG. 2 shows a health management manner according to an embodiment of this application;
FIG. 3 is a schematic diagram of an example of a hardware component scenario of a health management system according to an embodiment of this application;
FIG. 4A and FIG. 4B are a schematic diagram of an example of information interaction in a health management method according to an embodiment of this application;
FIG. 5 is a schematic diagram of examples of information categories in various types of user data and corresponding collection manners according to an embodiment of this application;
FIG. 6 is a schematic diagram of an example of an information flow direction in a process of generating a user health risk factor according to an embodiment of this application;
FIG. 7 is a schematic flowchart of an example of a user health risk factor recognition method according to an embodiment of this application;
FIG. 8 is a schematic diagram of an example of a health risk factor screening manner according to an embodiment of this application;
FIG. 9 is a schematic diagram of an example of an information flow direction of generating a personalized intervention plan according to an embodiment of this application;
FIG. 10 is a schematic diagram of an example of type classification in an intervention plan according to an embodiment of this application;
FIG. 11 is a schematic diagram of an example of an information flow direction in a training process of a benefit prediction model according to an embodiment of this application;
FIG. 12 is a schematic diagram of an example of a training process of a basic group model according to an embodiment of this application;
FIG. 13 is a schematic flowchart of an intervention plan benefit prediction method according to an embodiment of this application;
FIG. 14 is a schematic diagram of an example of delivering intervention plans of different types to different devices according to an embodiment of this application;

FIG. 15 is a schematic diagram of an example of an information flow direction in a process of generating an intervention plan effect assessment according to an embodiment of this application;

FIG. 16 is a schematic flowchart of an intervention plan effect assessment according to an embodiment of this application;

FIG. 17 is a schematic diagram of an example of an information flow direction in a user age estimation process according to an embodiment of this application;

FIG. 18 is a schematic flowchart of a user age estimation method according to an embodiment of this application;

FIG. 19 is a schematic diagram of an information flow direction of an intervention plan effect assessment according to an embodiment of this application;

FIG. 20 is another schematic flowchart of an intervention plan benefit prediction method according to an embodiment of this application;

FIG. 21 is a schematic diagram of an example of an information flow direction in another process of generating an intervention plan effect assessment according to an embodiment of this application;

FIG. 22 is a schematic flowchart of another intervention plan effect assessment method according to an embodiment of this application;

FIG. 23 is a schematic diagram of an example of a software module architecture of a health management system according to an embodiment of this application; and

FIG. 24 is a schematic diagram of an example of a hardware structure of an electronic device according to an embodiment of this application.

## DESCRIPTION OF EMBODIMENTS

Terms used in the following embodiments of this application are merely intended to describe specific embodiments, but are not intended to limit this application. As used in the specification of this application and the appended claims, the singular expression "a", "an", "the", "the foregoing", "such a", or "this" is also intended to include a plural expression unless otherwise clearly indicated in the context. It should also be understood that, the term "and/or" used in this application indicates and includes any or all possible combinations of one or more associated listed items.

The following terms "first" and "second" are merely intended for a purpose of description, and shall not be understood as an indication or implication of relative importance or implicit indication of a quantity of indicated technical features. Therefore, a feature limited by "first" or "second" may explicitly or implicitly include one or more features. In the descriptions of embodiments of this application, unless otherwise specified, "a plurality of" means two or more than two.

Health management involves many areas, for example, health risk assessment, health intervention, and health data monitoring. Health management is also implemented in various manners.

In a health management implementation, based on a large amount of epidemiological research, risk factors and risk assessment scales related to a single disease are provided in a disease control and prevention guideline. This provides a basis for a developed single disease risk assessment tool. However, in this health management manner, there is a lack of a method for recognizing and assessing an overall health status of a user. A single disease risk assessment cannot be associated with risk factors related to normal causes of death, cannot directly derive impact on mortality, and has a lack of measurement of an overall health risk level.

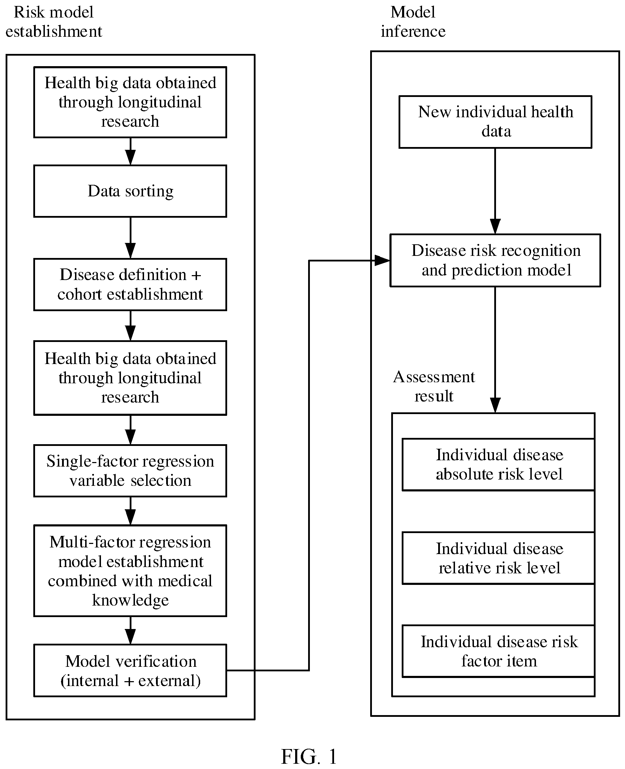

For example, in a disease risk assessment manner shown in FIG. 1, a chronic disease risk that affects health is mainly assessed and predicted. A specific process is as follows: obtaining health big data through longitudinal research, sorting the data, defining a threshold range of each indicator of a disease based on a disease name, and establishing a corresponding disease cohort by using statistical analysis software based on the threshold range of each indicator of the disease. After the cohort is established, a Cox single-factor regression analysis method is used to perform variable selection on each variable. Finally, multi-factor regression analysis is performed on a variable included in a Cox regression model, to construct a Cox proportional risk model. The Cox proportional risk model is verified internally and externally, to obtain a disease risk recognition and prediction model. New individual sign indicators and basic information are input, and a disease risk assessment result is generated based on a disease prediction model and disease risk assessment hyperbola.

However, in this disease risk assessment manner, the Cox proportional risk model is mainly constructed based on longitudinal follow-up research of a single disease; or implementation of some risk assessment tools is directly based on the risk assessment scales (for example, the China Diabetes Risk Score Scale) provided in the disease control and prevention guideline. The following problems may occur in public health management:

(1) A risk assessment model and method for a single disease is used for a risk level and a risk factor obtained based on the single disease, and pathogeneses and influencing factors of various diseases vary greatly. Although an assessment result can prevent chronic diseases with high prevalence such as cardio-cerebral vascular diseases and diabetes, the assessment result cannot reflect an overall health status of a user, a complete set of risk factors that affect health, and impact of risk factors on health and a lifespan of the user. As a result, an assessment of the overall health status of the user is inaccurate, and intervention measures to be taken are inaccurate or inappropriate. (2) This model is usually established based on small-scale follow-up research, with significant differences in population characteristics. In other populations, this assessment model algorithm has low accuracy due to differences in regions, ages, genders, and the like.

In another health management implementation, after a health assessment is performed, many intervention measures may be used, including exercise intervention, diet intervention, and the like. Most of the intervention measures are based on extensive recommendations from authoritative organizations for the whole population. For example, the World Health Organization recommends that adults aged 18 to 64 need to engage in at least 150 to 300 minutes of moderate-intensity aerobic exercise per week, and the Chinese Nutrition Society recommends avoiding or controlling diets high in oil, salt, and sugar.

However, because individual characteristics vary widely, these solutions are not targeted, and it is difficult for the user to control an appropriate amount.

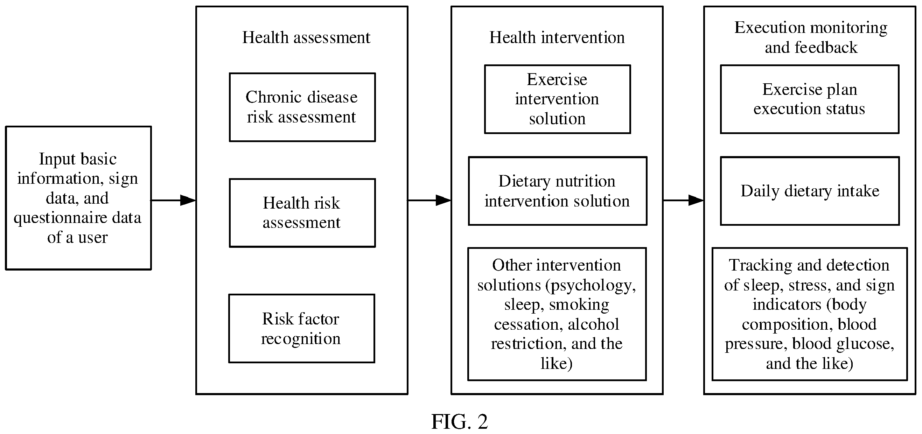

For example, a health management manner shown in FIG. 2 mainly includes health promotion and chronic disease management, and a process includes three parts:

(1) health assessment and risk assessment: first obtaining basic information of a user, collecting data including a symptom of discomfort, an exercise habit, a daily physical activity, a lifestyle, and the like of the user through standard questionnaires, collecting necessary sign and physical examination data (body composition, blood pressure, blood glucose, and blood lipid), assessing an individual disease risk level by using a disease risk assessment model, and recognizing a disease risk factor;

(2) health intervention: formulating a personalized intervention measure and a personalized implementation plan, for example, an exercise plan (mode, frequency, intensity, and time), for controllable risk factors (which are mostly bad lifestyles and behavior habits) based on risk factors corresponding to a chronic disease risk of an assessed object and a risk of a disease high-risk population; and

(3) execution monitoring and feedback: pushing the plan to the assessed object, monitoring a plan implementation status by using a wearable device or providing feedback on a plan implementation status through user subjective experience, and tracking changes of daily detectable sign indicators.

However, this health management manner is mainly focused on chronic disease management. In a process of assessing and generating an intervention policy, and implementing and monitoring an intervention plan, the following problems still exist:

(1) In this health management manner, an intervention plan can be only formulated based on a previous user input and related information, and a next intervention plan cannot be accurately and dynamically adjusted. As a result, an overall intervention effect is poor, or an intervention plan formulated later cannot be implemented.

(2) This health management manner is mainly focused on chronic disease management. However, in this case, some structural lesions are irreversible, and an intervention effect of the adopted intervention plan is greatly reduced in these populations.

In these health management manners, there is no health benefit prediction before the intervention plan is implemented and no comprehensive assessment and interpretation of an effect after the intervention plan is actually implemented. Adjustment of the intervention plan lacks an effective input, and closed-loop intervention management cannot be implemented. As a result, an effect on long-term improvement of user health is not significant. Consequently, user experience is poor and loyalty is low, and it is difficult to form real active health management.

This application provides another health management method. User sign data and daily behavior data may be used to help a user assess health risk factors currently exposed, assess overall health risk factors of the user, provide a personalized comprehensive intervention management solution for controllable risk factors closely related to individual health, and predict health benefits of a target plan. After a phase of an intervention plan is implemented, an intervention effect assessment and exercise diet joint assessment method may be further provided, to promote development of a healthy life of the user and achievement of an active health management objective.

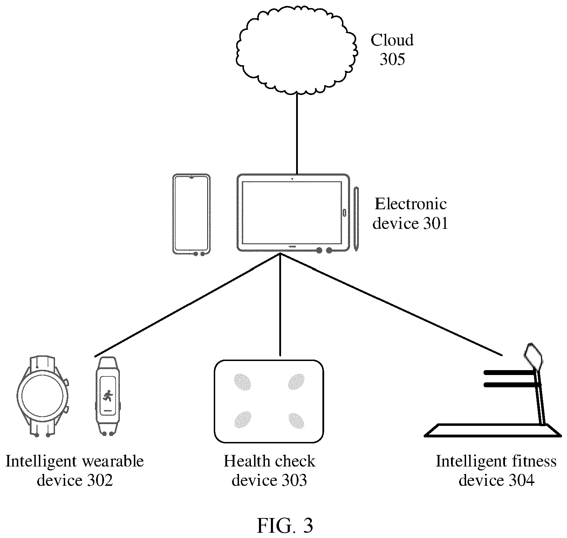

The health management method in this application is applied to a health management system. FIG. 3 is a schematic diagram of an example of a hardware component scenario of the health management system. The example health management system 300 may include an electronic device 301 (for example, a mobile phone or a tablet computer), an intelligent wearable device 302 (for example, a smart band or a smart wristwatch), a health check device 303 (for example, a body fat scale, a sphygmomanometer, a blood glucose meter, or a blood lipid monitor), and an intelligent fitness device 304 (for example, a smart treadmill or a smart rower). The health management system may include a cloud 305 including cloud infrastructure (for example, a cloud server).

The electronic device 301 is mainly responsible for collecting basic user information, health questionnaire information, and behavior data, synchronizing data of other devices in the health management system, and completing storage and processing; and is also responsible for assessing a user health risk, generating an intervention solution, predicting a health benefit, assessing an execution effect, and uploading user authorized data to the cloud 305.

The intelligent wearable device 302 is mainly responsible for monitoring and collecting exercise, sleep, and stress data of a user.

The health check device 303 is mainly responsible for detecting and collecting health indicators (body composition, blood pressure, blood glucose, and blood lipid) of the user.

The intelligent fitness device 304 may receive an exercise solution sent by the electronic device 301 and automatically execute the exercise solution, and may return exercise data to the electronic device 301 after the execution is completed.

The cloud 305 is mainly responsible for training a basic group model for health benefit prediction, managing and updating an intervention knowledge base, and the like.

It may be understood that, in actual application, the health management system 300 may include more or fewer devices. This is not limited herein.

The following describes the health management method according to embodiments of this application with reference to the example health management system 300.

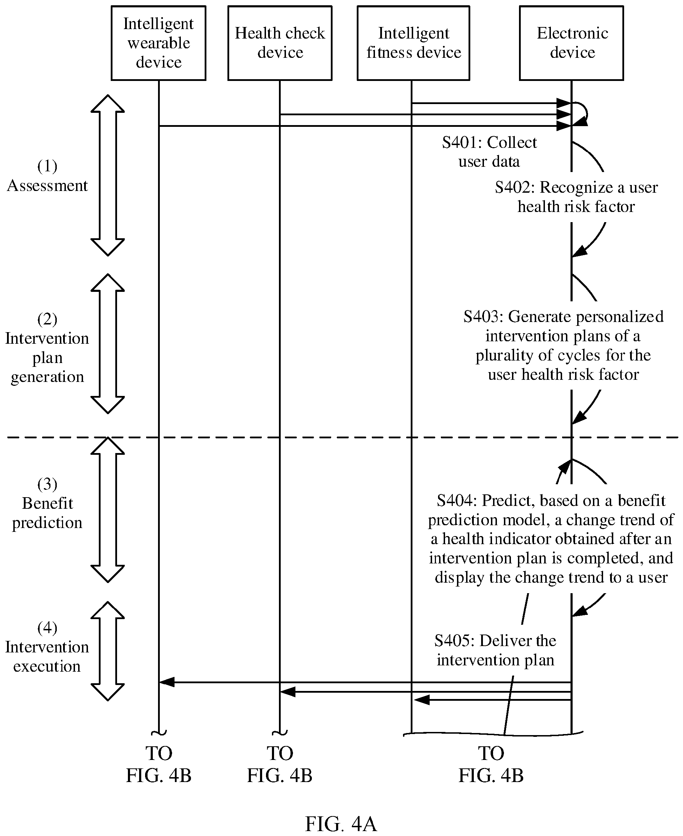

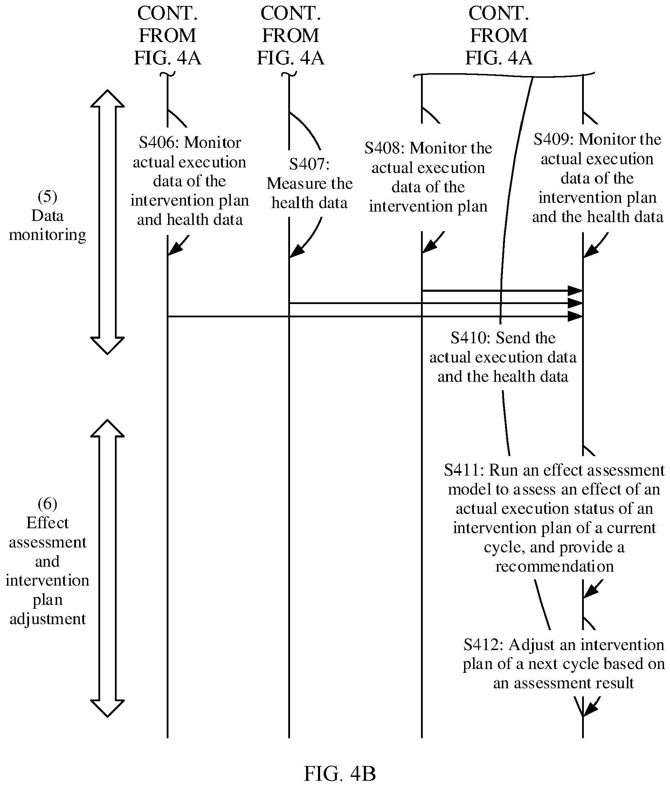

FIG. 4A and FIG. 4B are a schematic diagram of an example of information interaction in a health management method according to an embodiment of this application. For ease of understanding, the health management method in this embodiment of this application may be divided into six phases.

(1) An assessment phase includes steps S401 and S402: The electronic device 301 automatically assesses and recognizes, from overall health risk factors based on user data, a controllable health risk factor that is of a user and that is currently exposed, to obtain a user health risk factor.

(2) An intervention plan generation phase includes step S403: The electronic device 301 generates personalized intervention plans of a plurality of cycles for the recognized user health risk factor. For example, a 3-month intervention plan may be generated, where every 7 days in the intervention plan are a cycle.

(3) A benefit prediction phase includes step S404: The electronic device 301 predicts, based on a prediction benefit model, a change trend of a health indicator obtained after the intervention plan is completed, and displays the change trend to the user.

(4) An intervention execution phase includes step S405: The electronic device 301 delivers the intervention plan to another device in the health management system 300, to execute the intervention plan.

(5) A data monitoring phase includes steps S406 to S410: Each device in the health management system 300 monitors actual execution data and/or a value of the health indicator in an execution process of the intervention plan, and summarizes the actual execution data and/or the value of the health indicator to the electronic device 301.

(6) An effect assessment and intervention plan adjustment phase includes steps S411 and S412: The electronic device 301 assesses an execution effect of an intervention plan of a current cycle, and adjusts an intervention plan of a next cycle accordingly.

The following describes the steps in each phase in detail.

(1) Assessment phase:
S401: Each device in the health management system collects the user data, and summarizes the user data to the electronic device.

For ease of description, in this application, the user data may be classified as follows:

basic user information: including data of deterministic basic user information;
user behavior data: including data related to a user behavior; and
user health data: including data related to a user health status.

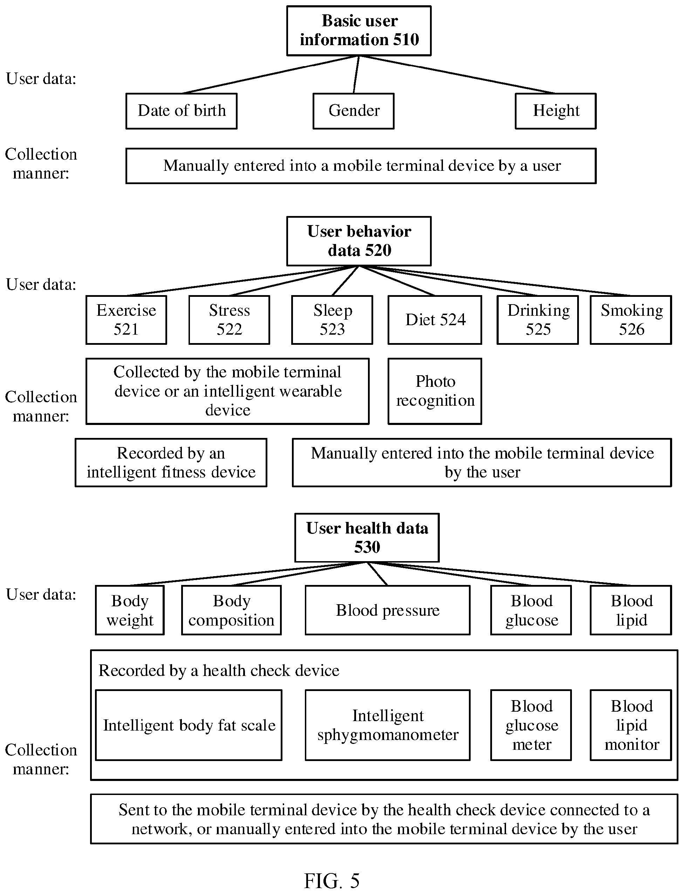

FIG. 5 is a schematic diagram of examples of information categories in various types of user data and corresponding collection manners according to an embodiment of this application.

For example, for basic user information 510:
In some embodiments, the basic user information may include a date of birth (age) and/or a gender of the user. In some embodiments, the basic user information may further include information such as a height. This is not limited herein.

In some embodiments, the basic user information may be directly and manually entered into the electronic device 301 by the user. In some embodiments, the electronic device 301 may directly recognize and extract the basic user information based on data stored in the electronic device 301 by the user, account information filled in by the user, and the like. This is not limited herein.

For example, for user behavior data 520:
In some embodiments, the user behavior data may include at least one of exercise data 521, stress data 522, sleep data 523, diet data 524, drinking data 525, and smoking data 526 of the user.

For the exercise data 521:
In some embodiments, the exercise data may be collected by the electronic device 301 or the intelligent wearable device 302. In some embodiments, the exercise data may alternatively be recorded by the intelligent fitness device, and then sent to the electronic device 301 through a network, or manually entered into the electronic device 301 by the user.

It may be understood that various types of data in the user behavior data may further include more subdivided data on different aspects.

For example, the exercise data may include data about exercise duration, an exercise step count, an exercise distance, an exercise heart rate, an exercise intensity level, a physical activity level, and the like.

The electronic device 301 or the intelligent wearable device 302 that has a positioning function and that has an accelerometer and a gyroscope sensor may collect the exercise duration, the exercise step count, and the exercise distance of the user.

The intelligent wearable device 302 with a photoplethysmography (photoplethysmography, PPG) heart rate monitoring function may collect a resting heart rate and an exercise heart rate $HRe$ of the user. Herein, PPG is a simple optical technology used to detect volumetric changes in blood in peripheral vascular circulation. This is a low-cost and non-invasive method that may be used to perform measurements at the surface of the skin, and is widely used in wearable devices for heart rate monitoring.

After the exercise heart rate $HRe$ of the user is collected, with reference to the American college of sports medicine (American college of sports medicine, ACSM) guidelines, the electronic device 301 or the intelligent wearable device 302 may estimate a maximum heart rate $HRmax$ of the user by subtracting the age from 220. Whether the exercise intensity level of the user is low-intensity exercise, moderate-intensity exercise, or high-intensity exercise may be calculated based on the maximum heart rate $HRmax$ and the exercise heart rate $HRe$ of the user. For example, a calculation and grading method may be as follows: The low-intensity exercise is: $HRe < 65\% \times HRmax$; the moderate-intensity exercise is: $65\% \times HRmax \le HRe \le 75\% \times HRmax$; and the high-intensity exercise is: $HRe > 75\% \times HRmax$.

Based on the exercise duration and exercise intensity of the user, the electronic device 301 or the intelligent wearable device 302 may obtain an exercise amount and a physical activity level (low, medium, or high) of the user in recent seven days by using a calculation method of the physical activity level in the international physical activity questionnaire (international physical activity questionnaire, IPAQ).

For the stress data 522:
A daily stress value of the user may be collected by using the intelligent wearable device 302.

For the sleep data 523:
The intelligent wearable device 302 based on a PPG signal analysis technology or the electronic device 301 based on an ultrasonic respiratory signal analysis technology may collect sleep stages and sleep quality data.

The electronic device 301 or the intelligent wearable device 302 may further receive night sleep start time manually recorded by the user, and may determine, by comparing fall-asleep time manually recorded by the user with fall-asleep time of the user recognized by the device, whether the user has difficulty in falling asleep and a bad habit of going to bed late.

In some embodiments, the sleep data may alternatively be a sleep status of the user that is manually entered into the electronic device 301 by the user.

For the diet data 524:
The diet data may be generated when the user performs photo recognition during eating, or may be manually entered into the electronic device 301 by the user based on a diet status of the user. Specifically, a dietary intake collected by the electronic device 301 through manual entering or photo recognition by the user may include a food type, an intake amount, and the like. The electronic device 301 may further obtain, based on a nutrient element proportion of each ingredient in a diet library, a nutrient element ingested by the user.

For the drinking data 525 and the smoking data 526:
The drinking data 525 and the smoking data 526 may be manually entered into the electronic device 301 by the user after the user drinks alcohol or smokes.

For example, the electronic device 301 may collect smoking and drinking habits (frequency and average amount per time) of the user by using a questionnaire.

For example, for user health data 530:
In some embodiments, the user health data may include at least one of a body weight, body composition, blood pressure, blood glucose, and blood lipid.

The user health data may be recorded by the health check device 303, and then sent to the electronic device 301 through the network, or may be manually entered into the electronic device 301 by the user. This is not limited herein.

For example, the user may use an intelligent body fat scale to collect weight and body composition data per week.

For example, the electronic device 301 may further obtain a body mass index BMI by dividing weight (kg) by a square of height (m$^2$). Further, the electronic device 301 may classify the body weight of the user as lean (BMI < 18.5), normal (18.5 ≤ BMI ≤ 24), overweight (24 < BMI ≤ 28), or obese (BMI > 28).

For example, the user may collect systolic blood pressure and diastolic blood pressure by using an intelligent sphygmomanometer per day, to obtain blood pressure data.

For example, the user may collect fasting plasma glucose FPG by using an intelligent blood glucose meter per day, to obtain blood glucose data.

For example, the user may collect blood lipid indicators (total cholesterol, triglycerides, low density lipoprotein cholesterol, and high density lipoprotein cholesterol) by using a blood lipid monitor per week, to obtain blood lipid data.

For example, the user may manually enter or upload a picture of a latest physical examination report (where specific content is recognized by using an OCR technology) to the electronic device 301, and the electronic device 301 may obtain physical examination information of the user.

In addition, the electronic device 301 may further collect a personal disease history, a family disease history, and the like of the user by using a questionnaire.

It may be understood that, in the foregoing data collection process, that the user manually enters data into the electronic device 301 is not limited to a form in which the user directly enters the related data, and may further include forms in which the electronic device 301 displays a related data option for the user to select, the electronic device 301 displays a questionnaire for the user to fill in, the electronic device 301 records a check-in status of the user by using a check-in task, and the like, where manual intervention by the user is required to enable the electronic device 301 to obtain the related data. This is not limited herein.

After the another device (the intelligent wearable device 302, the health check device 303, and/or the intelligent fitness device 304) in the health management system collects related user data including the user behavior data and the user health data, the another device may upload and synchronize the collected data to the electronic device 301 through the network (for example, through a Bluetooth connection or a wireless network).

S402: The electronic device recognizes the user health risk factor based on the collected user data. After obtaining the user data collected by each device in the health management system 300, the electronic device 301 may recognize, based on the user data and a complete set of risk factors that affect health, a user health risk factor that is related to a lifestyle of the user and that is controllable. Specifically, the user health risk factor may be recognized in a plurality of different manners. The following describes in detail a user health risk factor recognition method according to an embodiment of this application by using one of the manners as an example.

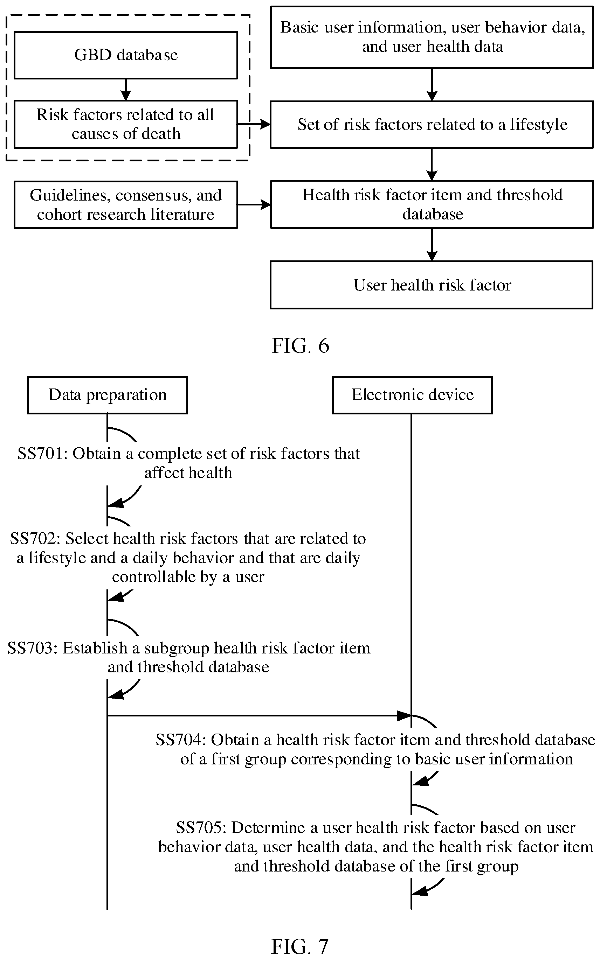

FIG. 6 is a schematic diagram of an example of an information flow direction in a process of generating a user health risk factor according to an embodiment of this application.

FIG. 7 is a schematic flowchart of an example of a user health risk factor recognition method according to an embodiment of this application.

The user health risk factor recognition method includes the following:

A process of establishing a subgroup database: Steps SS701 to SS703 are used to prepare data for user health risk factor recognition. In some embodiments of this application, the subgroup database may also be referred to as a risk factor correspondence of a plurality of groups.

A risk factor recognition process: Steps SS704 and SS705 are used to determine the user health risk factor based on the subgroup database and the collected user data.

The steps in the two processes are separately described in detail below with reference to FIG. 6 and FIG. 7.

SS701: Obtain, based on a population health and disease burden database, a complete set of risk factors that affect health.

The population health and disease burden database may be a database that is released by some large international or national organizations and that is used to display global or national population health and disease burden, for example, may be a Global Burden of Disease (Global Burden of Disease, GBD) database, or some other databases of the same type.

The GBD database is used as an example. Specifically, a total of more than 60 risk factors of all death-related diseases may be obtained by querying the GBD database. In addition, common factors such as sleep and stress that may affect health and that are not counted in the database are added to obtain the complete set of risk factors that affect health.

SS702: Select, from the complete set of risk factors that affect health, health risk factors that are related to a lifestyle and a daily behavior and that are daily controllable by the user, to obtain a set of controllable risk factors related to life and behavior.

It may be understood that, in the complete set of risk factors that affect health obtained in step SS701, many factors are related to environmental pollution, genetics, and the like. These factors are difficult to collect in a home scenario, and are difficult to control at a personal level. Therefore, these factors need to be excluded. Therefore, controllable risk factors (which may be referred to as a set of controllable risk factors related to life and behavior) related to a lifestyle and a daily behavior are ultimately selected, and at least an exercise amount in recent 7 days, a daily dietary intake (fruits, vegetables, red meat, grains, and salt), a daily amount of drinking, a daily amount of smoking, a body fat percentage, systolic blood pressure, diastolic blood pressure, fasting plasma glucose, and a blood lipid indicator may be included.

There may be a plurality of specific screening manners in step SS702, for example, which risk factors are controllable may be determined based on various standards or rules, or which risk factors are related to a lifestyle and a daily behavior may be determined based on various standards or rules.

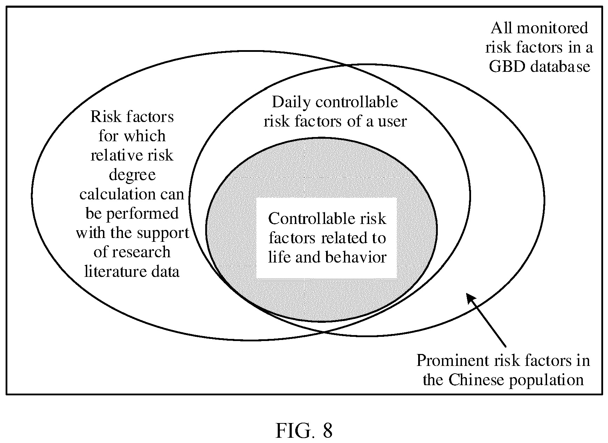

FIG. 8 is a schematic diagram of an example of a health risk factor screening manner according to an embodiment of this application. A set A of prominent risk factors in the Chinese population can be selected from all monitored risk factors in the GBD database, and a set B of risk factors for which relative risk degree calculation can be performed with the support of research literature data can be selected from all the monitored risk factors. Risk factors shared by the set A and the set B are selected, to obtain a set C of daily controllable risk factors of the user. Then, some risk factors related to the lifestyle and the daily behavior of the user are selected from the set C as the controllable risk factors related to life and behavior.

It may be understood that, in some embodiments, the health risk factors that are related to the lifestyle and the daily behavior and that are daily controllable by the user may be selected in another manner from the complete set of risk factors that affect health. This is not limited herein. SS703: Establish a subgroup health risk factor item and threshold database based on a set of controllable risk factors related to life and behavior.

Based on the risk factors selected in step SS702, a classification threshold of each risk factor affecting health can be obtained by searching, refining and assessing evidence such as guidelines, expert consensus, and literature. Therefore, a health risk factor database may be established, and a correspondence of the database may include risk factor categories and category classification criteria. Since some of these risk factors are classified by gender or age, the correspondence of the database may also include a gender and an age. If different genders and age ranges are considered as a different group, a health risk factor item and threshold database may also be referred to as the subgroup health risk factor item and threshold database.

Table 1 is a schematic table of an example of a subgroup health risk factor item and threshold database according to this embodiment of this application.

**Table 1**

| Gender | Age range | Risk factor | Risk range value |
|---|---|---|---|
| - | - | Insufficient physical activity | Physical activity level rated "low" |
| Male | ≤ 55 | High body fat percentage | > 20% |
| Male | > 55 | High body fat percentage | > 20% + (2% × (age - 55)/5)) |
| Female | ≤ 50 | High body fat percentage | > 30% |
| Female | > 50 | High body fat percentage | > 30% + (2% × (age - 50)/5)) |
| - | ≤ 35 | ... | ... |
| - | > 30 | ... | ... |
| Male | - | ... | ... |
| Female | - | ... | ... |
| ... | ... | ... | ... |
| - | - | Insufficient daily vegetable intake | Daily vegetable intake < 300 g |

In Table 1, "-" is used to indicate all genders or all age groups. In actual application, all genders or all age groups may be recognized in another form, for example, a specific value "0" or "1" is used. This is not limited herein.

It may be understood that Table 1 shows only an example of the subgroup health risk factor item and threshold database. In actual application, the subgroup health risk factor item and threshold database may be stored in another form. This is not limited herein. In some embodiments, the subgroup health risk factor item and threshold database may not store the risk range value corresponding to the risk factor, but a health range value corresponding to the risk factor.

In some embodiments, both the risk range value corresponding to the risk factor and the health range value corresponding to the risk factor may be stored. This is not limited herein.

After the subgroup health risk factor item and threshold database is created, the subgroup health risk factor item and threshold database may be stored on the cloud 305, or may be directly stored in the electronic device 301. This is not limited herein.

In some embodiments of this application, the health risk factor item and threshold database may also be referred to as a risk factor correspondence. Similarly, the subgroup health risk factor item and threshold database may also be referred to as a risk factor correspondence of a plurality of groups. Age ranges and/or genders corresponding to risk factor correspondences of different groups are different. A risk factor correspondence of one group includes a correspondence between one or more health risk factors and a corresponding preset condition of the one or more health risk factors. For example, in Table 1, a preset condition corresponding to the health risk factor "Insufficient physical activity" is "Physical activity level rated "low"". For another example, in the risk factor correspondence of a "Male, $\leq 55$" group, a preset condition corresponding to the health risk factor "High body fat percentage" is "> 20%".

Optionally, in some embodiments, for any health risk factor in the risk factor correspondence, a preset condition corresponding to the health risk factor may include a more refined preset subcondition, to present a risk degree of a health risk factor corresponding to the preset subcondition.

For example, in the risk factor correspondence of the "Male, $\leq 55$" group, the preset condition corresponding to the health risk factor "High body fat percentage" is "> 20%". The preset condition may further include three preset subconditions: mild: > 20% and $\leq 25\%$; ordinary: > 25% and $\leq 28\%$; and severe: > 28%.

For example, in the risk factor correspondence, a preset condition corresponding to the health risk factor "Insufficient daily vegetable intake" is "Daily vegetable intake < 300 g". The preset condition may further include two preset subconditions: insufficient: 250 g $\leq$ daily vegetable intake < 300 g; and severely insufficient: daily vegetable intake < 250 g.

SS704: Obtain a health risk factor item and threshold database of a first group corresponding to basic user information.

In some embodiments, the electronic device 301 may obtain, from the cloud 305 based on the basic user information collected in step S401 and the subgroup health risk factor item and threshold database stored on the cloud 305, the health risk factor item and threshold database of the first group corresponding to the basic user information.

In some embodiments, if the electronic device 301 stores the subgroup health risk factor item and threshold database, the electronic device 301 may determine, from the subgroup health risk factor item and threshold database based on the basic user information collected in step S401, the health risk factor item and threshold database of the first group corresponding to the basic user information.

For example, if the basic user information collected in step S401 is male, 40 years old, and 170 cm tall, and the subgroup health risk factor item and threshold database established in step SS703 is shown in Table 1, the health risk factor item and threshold database that is of the first group corresponding to the basic user information and that is obtained by the electronic device 301 may be shown in Table 2:

**Table 2**

| Gender | Age range | Risk factor | Risk range value |
|---|---|---|---|
| - | - | Insufficient physical activity | Physical activity level rated "low" |
| Male | $\leq 55$ | High body fat percentage | > 20% |
| - | > 30 | ... | ... |
| Male | - | ... | ... |
| ... | ... | ... | ... |
| - | - | Insufficient daily vegetable intake | Daily vegetable intake < 300 g |

For example, if the basic user information collected in step S401 is female, 32 years old, and 150 cm tall, and the subgroup health risk factor item and threshold database established in step SS703 is shown in Table 1, the health risk factor item and threshold database that is of the first group corresponding to the basic user information and that is obtained by the electronic device 301 may be shown in Table 3:

**Table 3**

| Gender | Age range | Risk factor | Risk range value |
|---|---|---|---|
| - | - | Insufficient physical activity | Physical activity level rated "low" |
| Female | ≤ 50 | High body fat percentage | > 30% |
| Female | > 50 | High body fat percentage | > 30% + (2% × (age - 50)/5)) |
| - | ≤ 35 | ... | ... |
| - | > 30 | ... | ... |
| Female | - | ... | ... |
| ... | ... | ... | ... |
| - | - | Insufficient daily vegetable intake | Daily vegetable intake < 300 g |

SS705: Determine the user health risk factor based on the user behavior data, the user health data, and the health risk factor item and threshold database of the first group.

After obtaining the health risk factor item and threshold database of the first group, the electronic device 301 may determine, based on the user behavior data and the user health data that are in the user data collected in step S401, a health risk factor that is of the user and that has been exposed, namely, the user health risk factor.

For example, if the health risk factor item and threshold database of the first group of the user is shown in Table 2, and the physical activity level obtained from the user behavior data in the user data collected in step S401 is "low", which meets the risk value range of the risk factor "Insufficient physical activity", the risk factor "Insufficient physical activity" is a health risk factor that is of the user and that has been exposed, and should be included in the user health risk factor of the user. For example, if the health risk factor item and threshold database of the first group of the user is shown in Table 2, and the body fat percentage in the user health data in the user data collected in step S401 is 25%, which is greater than 20% and meets the risk value range of the risk factor "High body fat percentage", the risk factor "High body fat percentage" is also a health risk factor that is of the user and that has been exposed, and should be included in the user health risk factor of the user.

For example, if the health risk factor item and threshold database of the first group of the user is shown in Table 2, and the daily vegetable intake in the user behavior data in the user data collected in step S401 is 150 g, which is less than 300 g and meets the risk value range of the risk factor "Insufficient daily vegetable intake", the risk factor "Insufficient daily vegetable intake" is also a health risk factor that is of the user and that has been exposed, and should be included in the user health risk factor of the user.

It should be noted that, the electronic device 301 may prestore or pre-obtain a correspondence between the health risk factor item and threshold database and data of different types in the user behavior data and the user health data. Therefore, the electronic device 301 may query, based on the collected user behavior data and user health data, the health risk factor item and threshold database of the first group to determine whether each risk factor is an exposed risk factor of the user, and add the risk factor to the user health risk factor of the user.

It may be understood that, in some embodiments, if the preset condition of the risk factor correspondence includes a more refined preset subcondition used to present a risk degree of the corresponding health risk factor, the electronic device may not only determine the user health risk factor based on the user behavior data, the user health data, and the risk factor correspondence of the first group, but also determine an exposure level (namely, a risk degree) of one or more of the user health risk factors.

The user health risk factor recognition method in this embodiment of this application can reflect an overall health risk status of the user, and expose the complete set of risk factors that affect health, so that a health status of the user can be managed more accurately.

(2) Intervention plan generation phase

S403: The electronic device generates personalized intervention plans of a plurality of cycles for the user health risk factor.

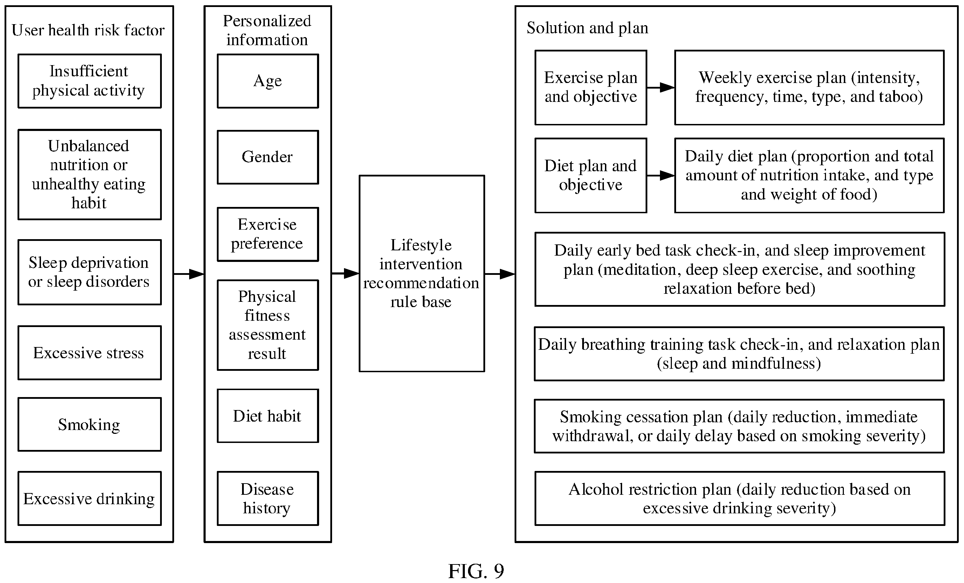

FIG. 9 is a schematic diagram of an example of an information flow direction of generating a personalized intervention plan according to an embodiment of this application. After the user health risk factor (for example, insufficient physical activity, unbalanced nutrition or an unhealthy eating habit, sleep deprivation or sleep disorders, excessive stress, smoking, or excessive drinking) is recognized in step S402, a specific intervention plan may be generated by using a lifestyle intervention solution recommendation rule base based on personalized information (for example, a user age, a gender, an exercise preference, a physical fitness assessment result, a diet habit, and a disease history) of the user obtained based on the collected user data.

For example, an exercise solution and a weekly exercise plan may be generated for insufficient physical activity.

For example, a diet solution and a daily diet plan may be generated for unbalanced nutrition or an unhealthy eating habit.

For example, a daily early bed check-in and a sleep improvement plan may be generated for sleep deprivation or sleep disorders.

For example, a daily breathing training check-in and a relaxation plan may be generated for excessive stress.

For example, a smoking cessation and alcohol restriction solution and plan may be generated for smoking and excessive drinking.

It may be understood that, which type of data in the user data collected in step S401 belongs to the personalized information of the user may be preset, or the electronic device may automatically recognize the personalized information of the user from the collected user data based on a preset algorithm. This is not limited herein.

Optionally, a rule in the lifestyle intervention recommendation rule base may be preset, or may be a type of deep learning algorithm. This is not limited herein.

For example, a manner of setting the lifestyle intervention recommendation rule base may be as follows:

<1> A type of an exercise solution and a diet solution is determined based on a current body mass index (body mass index, BMI), blood pressure indicator, blood glucose indicator, and blood lipid indicator of the user and by querying a past medical history database of the user. The type is as follows: body weight management (BMI > 24 kg/m2), blood pressure management (systolic blood pressure SBP > 130 mmHg, or diastolic blood pressure DBP > 80 mmHg, or hypertension is diagnosed), or blood glucose management (fasting plasma glucose (fasting plasma glucose, FPG) > 6.1 mmol/L, or diabetes is diagnosed). If indicators of the user are within a normal range and the user has no history of hypertension or diabetes, the type of the exercise solution and the diet solution is health promotion. If a plurality of types are met, priorities of the determined types of the exercise solution and the diet solution are sorted as: blood glucose management, blood pressure management, body weight management, and health promotion.

<2> An objective of each exercise and diet solution type is to achieve a normal value for a corresponding indicator, and an objective of the solution type of health promotion is to maintain health (sufficient exercise and balanced diet and nutrition).

<3> An exercise intervention knowledge base for body weight management, an exercise intervention knowledge base for blood pressure management, an exercise intervention knowledge base for blood glucose management, and an exercise intervention knowledge base for health promotion are established by searching evidence such as guidelines, expert consensus, and literature for the type and objective of each exercise solution and diet solution. With reference to the standard of Chinese dietary reference intakes, a diet recommendation knowledge base for body weight management, a diet recommendation knowledge base for blood pressure management, a diet recommendation knowledge base for blood glucose management, and a diet recommendation knowledge base for health promotion are established.

<4> An exercise plan and solution are generated for exercise by using an exercise intervention knowledge base based on the type and objective of each exercise solution and diet solution, and a user exercise preference and physical fitness, and may include: an exercise type, exercise frequency, exercise intensity (reflected as an exercise heart rate range), exercise time, an exercise taboo, and the like.

<5> A daily diet plan can be generated for a diet by using a diet recommendation knowledge base based on the type and objective of each exercise solution and diet solution, and an eating habit of the user, including a recommended daily energy intake per meal, a proportion and total amount of nutrition intake, a type and weight of food, a food recommendation, a type of food to avoid or a food taboo, and the like.

<6> For going to bed late (later than 23:00) or severe sleep deprivation (< 6 hours), two check-in tasks: go to bed early and get up regularly can be generated, a sleep improvement plan (for example, meditation, deep sleep exercise, and soothing relaxation before bed) can be generated, and a sleep aid music service can be further provided to help the user improve sleep disorders and develop a normal work and rest schedule.

<7> For excessive stress, a daily breathing training check-in task can be generated, and sleep and mindfulness relaxation services can be provided.

<8> For bad habits (smoking and alcohol abuse), a smoking cessation plan (for example, daily reduction or immediate withdrawal, or daily delay) and a smoking cessation reminder check-in task can be generated based on frequency of smoking and an amount of smoking each time; and an alcohol restriction plan (for example, daily reduction) and an alcohol restriction reminder check-in task can be generated based on frequency of drinking and an amount of drinking each time. In addition, messages about harm caused by the bad habits such as smoking and alcohol abuse can be pushed weekly, to improve education of such a population.

It may be understood that, if the electronic device also determines exposure levels of one or more health risk factors when determining the health risk factors of the user, the exposure levels of the health risk factors may also be added to input data for generating the intervention plan by using the exposure levels of the health risk factors as a reference for generating the intervention plan. Therefore, a more targeted intervention plan is generated for a health risk factor with a higher risk degree.

Specifically, the electronic device may extract personalized user information from the user data collected in step S401, and then query the lifestyle intervention recommendation rule base based on the personalized user information and the user health risk factor recognized in step S402, to obtain a first intervention plan. The first intervention plan may include a first exercise plan, a first diet plan, and a first health habit check-in task set.

The first intervention plan may include intervention solutions of a plurality of cycles. For example, the first intervention plan may include a three-month intervention solution, where a weekly (7-day) intervention solution is one cycle, and the first intervention plan includes 12 cycles of intervention solutions.

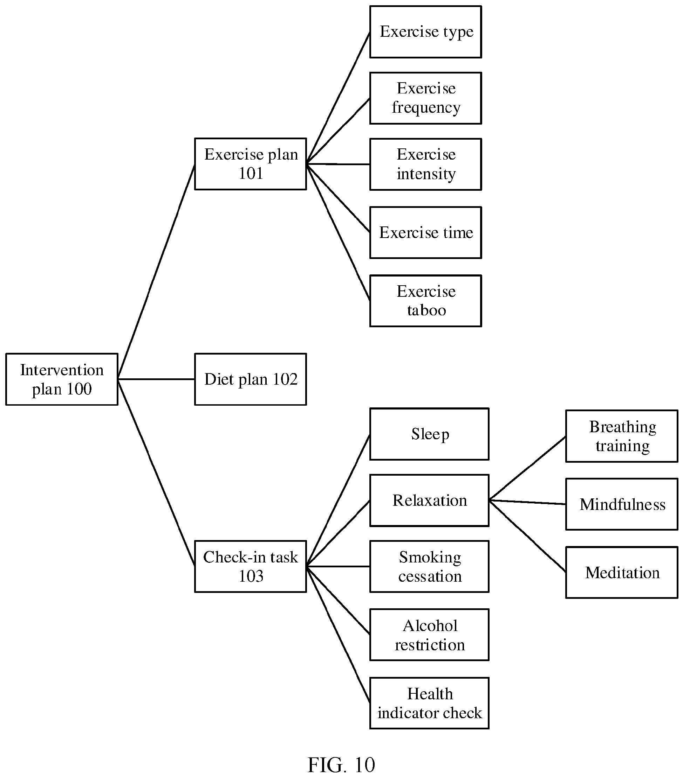

It should be noted that the generated intervention plan may include a plurality of plans of different types. For example, FIG. 10 is a schematic diagram of an example of type classification in an intervention plan according to an embodiment of this application. An intervention plan 100 may be divided into an exercise plan 101, a diet plan 102, and a check-in task 103.

The exercise plan 101 may be used to plan exercise in a period of time.

For example, an exercise type, exercise frequency, exercise intensity, exercise time, an exercise taboo, and the like may be included.

In some embodiments, the exercise plan 101 may further include exercise solution configuration information of the intelligent fitness device, and the intelligent fitness device may directly run based on the exercise solution configuration information, without requiring manual configuration by the user.

It may be understood that content in the exercise plan 101 may be generally monitored by the intelligent wearable device or the electronic device when the user actually performs the exercise plan 101, or the intelligent fitness device may record some execution processes or execution results of the exercise plan 101.

The diet plan 102 may be used to plan a diet in a period of time.

For example, a recommended daily energy intake per meal, a proportion and total amount of nutrition intake, a type and weight of food, a food recommendation, a type of food to avoid or a food taboo, and the like may be included.

The check-in task 103 may be used to plan a health event whose data cannot be directly monitored by the electronic device in a period of time.

For example, a sleep check-in task, a relaxation check-in task, a smoking cessation check-in task, an alcohol restriction check-in task, a health indicator check check-in task, and the like may be included.

Specifically, the sleep check-in task may include a sleep reminder check-in and a getting up check-in; the relaxation check-in task may include a breathing training check-in, a mindfulness training check-in, a meditation check-in, and the like; the smoking cessation check-in task may include a daily non-smoking check-in; the alcohol restriction check-in task may include a daily non-drinking check-in; the health indicator check check-in task may include a scheduled reminder for a health indicator check check-in; and the like.

In the intervention plan generation phase in this embodiment of this application, a personalized and achievable health intervention plan is provided based on a user habit and a group characteristic, and a wearable device and a portable home check device are fully utilized to track a user behavior and a health indicator. This provides an effective input for health benefit assessment and effect assessment in the health management system.

(3) Benefit prediction phase

S404: The electronic device predicts, based on the benefit prediction model, the change trend of the health indicator obtained after the intervention plan is completed, and displays the change trend to the user.

The electronic device may predict, based on the benefit prediction model, a predicted value of the health indicator obtained after the intervention plan is completed.

After generating the personalized intervention plans of the plurality of cycles, the electronic device may predict, based on the benefit prediction model, a change trend of the health indicator obtained after a part and/or all of the intervention plan is completed, and display the change trend to the user.

Optionally, the change trend may be in a text form, or may be in a chart form, or may be in an animation form, or the like. This is not limited herein.

Optionally, the change trend of the health indicator obtained through prediction by the electronic device after the part and/or all of the intervention plan is completed includes predicted values of the health indicator obtained after a part of and/or all cycles of the intervention plans of the plurality of cycles are separately completed.

For example, for the first intervention plan whose total duration is 3 months and having 12 cycles in total, the electronic device may separately predict predicted values of the health indicator obtained after the 12 cycles of the intervention plan are separately completed. Then, a change trend of the predicted values of the 12 health indicators is displayed in a line chart. Alternatively, predicted values of the health indicator after first six cycles of the intervention plan are separately completed may be obtained through prediction, and a change trend of the predicted values of the six health indicators is displayed to the user in a line chart. This is not limited herein.

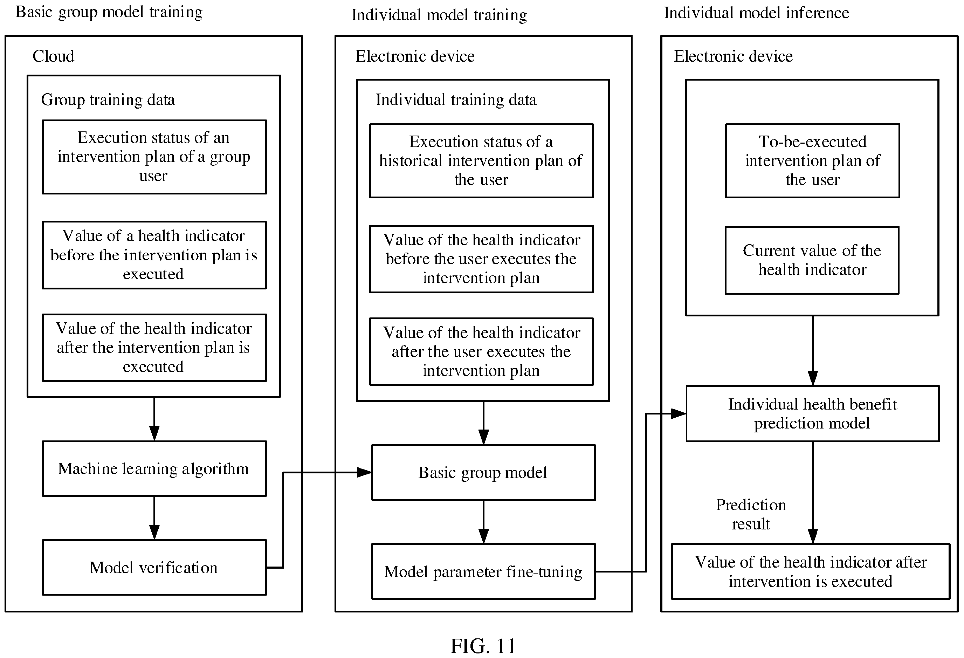

FIG. 11 is a schematic diagram of an example of an information flow direction in a training process of a benefit prediction model according to an embodiment of this application. To complete this process, two health benefit prediction models need to be established by using a machine learning algorithm. One is a basic group model established on a cloud, and the other is an individual health benefit prediction model established in an electronic device.

A process of establishing the basic group model may be as follows:

<1> With knowledge and consent of a user, user data, an intervention plan, an intervention plan execution status, a value of a health indicator before the intervention plan is executed, and a value of the health indicator after the intervention plan is executed that are on the electronic device are anonymized and then uploaded to the cloud.

<2> A large amount of data uploaded to the cloud by electronic devices belonging to different users is sorted and cleaned, and is divided into group training data for different groups based on age groups and genders.

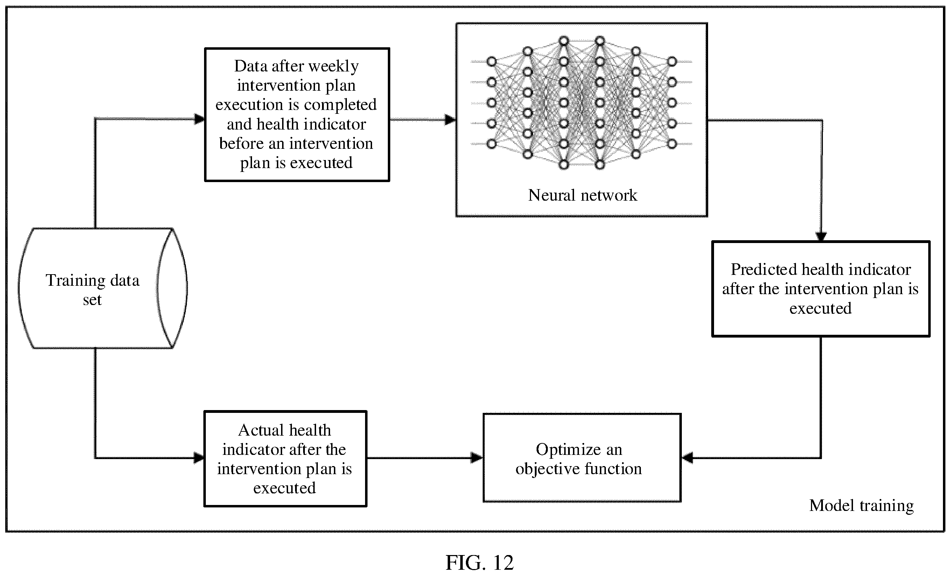

<3> FIG. 12 is a schematic diagram of an example of a training process of a basic group model.

Group training data for a group can be divided into a training data set and a validation data set, and the basic group model can be supervised by using a machine learning neural network or a regression algorithm. An independent variable is a weekly intervention plan execution status (average daily exercise duration, an exercise heart rate, a dietary calorie intake, three nutrient intakes, sleep duration, an amount of smoking and an amount of drinking, or the like) of a user in the group, and a value of a health indicator (a body weight, BMI, a body fat percentage, systolic blood pressure, diastolic blood pressure, fasting plasma glucose, total cholesterol, triglyceride, or the like) before an intervention plan is executed. A dependent variable is a value of the health indicator after a part and/or all of the intervention plan is executed. An objective function of the machine learning neural network or the regression algorithm may be a mean square error of values of the health indicator before and after the intervention plan is executed.

The objective function may be optimized by using an actual value of the health indicator obtained after the intervention plan in the training data set is executed, so that a difference between a predicted value of the health indicator obtained by using the machine learning neural network or the regression algorithm after the intervention plan is executed and the actual value of the health indicator obtained after the intervention plan is executed is within a preset range, to obtain a to-be-verified basic group model.

Then, data in the validation data set may be used to verify the to-be-verified basic group model. If the difference between the predicted value of the health indicator obtained after the intervention plan is executed and the actual value of the health indicator obtained after the intervention plan is executed is within the preset range, it can be determined that the basic group model of the group is obtained.

It may be understood that, for different groups, different basic group models may be obtained through training. This is not limited herein.

An individual health benefit prediction model of the user may be established based on the basic group model, and an establishment process may be as follows:

<1> A basic group model of a first group matching basic user information of an electronic device is obtained from a cloud.

<2> An execution status of a historical intervention plan of the user, the value of the health indicator before the user executes the intervention plan, and the value of the health indicator after the user executes the part and/or all of the intervention plan that are in the electronic device are used as individual training data of the user, to train the obtained basic group model of the first group, and fine-tune parameters of the basic group model, so that the individual health benefit prediction model of the user can be obtained.

With reference to the foregoing process of establishing the basic group model and the individual health benefit prediction model, the following describes in detail an intervention plan benefit prediction method according to an embodiment of this application.

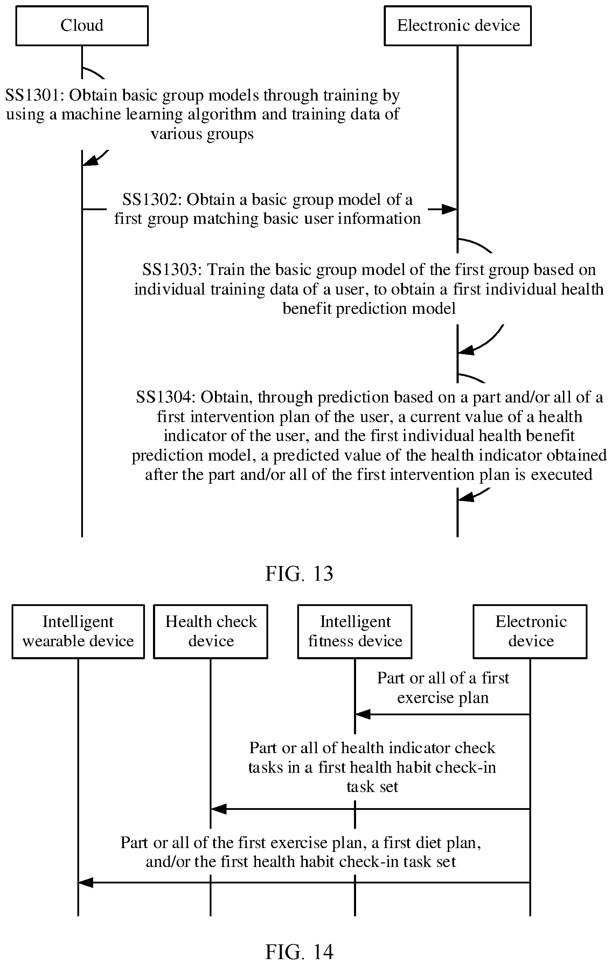

FIG. 13 is a schematic flowchart of an intervention plan benefit prediction method according to an embodiment of this application.

SS1301: Obtain basic group models through training by using a machine learning algorithm and training data of various groups.

As described in the foregoing process of establishing the basic group model, a cloud may obtain the basic group models through training by using the machine learning algorithm and the collected training data of various groups.

It may be understood that gender and age classification of each group may be consistent with that of different groups in step SS703, or may be different from that of different groups in step SS703. This is not limited herein.

For example, Table 4 shows an example of a plurality of different basic group models obtained through training.

**Table 4**

| Gender | Age | Basic group model |
|--------|-----|-------------------|
| Male | ≤ 18 | Basic group model A |
| Male | > 18 and ≤ 30 | Basic group model B |
| Female | ≤ 18 | Basic group model C |
| Female | > 18 and ≤ 30 | Basic group model D |
| ... | ... | ... |

In some embodiments, the basic group models may alternatively be an overall model, and the gender and the age are two variables. When the two variables are input, the overall model may be used as a basic group model of a specific group. This is not limited herein.

It may be understood that, in some embodiments, step SS1301 is performed by a cloud server. In some embodiments, step SS1301 may alternatively be performed by an electronic device after obtaining related data from a cloud server. This is not limited herein.

SS1302: Obtain a basic group model of a first group matching basic user information.

In some embodiments, if the basic group model is a basic group model of different groups classified based on the age and the gender shown in Table 4, the electronic device may obtain, based on the basic user information collected in step S401, the basic group model of the first group matching the basic user information.

For example, if a basic group model of a plurality of groups is shown in Table 4, and in the basic user information, the gender is male, and the age is 25 years old, the electronic device may obtain the basic group model B matching the information, and use the basic group model B as the basic group model of the first group to which the user belongs.

For example, if a basic group model of a plurality of groups is shown in Table 4, and in the basic user information, the gender is female, and the age is 13 years old, the electronic device may obtain the basic group model C matching the information, and use the basic group model C as the basic group model of the first group to which the user belongs.

SS1303: Train the basic group model of the first group based on individual training data of the user in the electronic device, to obtain a first individual health benefit prediction model.

The individual training data of the user in the electronic device may include completion data of a historical intervention plan of the user, health indicator data before the user executes an intervention plan, and health indicator data after the user executes the intervention plan.

It may be understood that, if the electronic device is a new electronic device, and there is no individual training data of the user, the basic group model of the first group may be directly used as the first individual health benefit prediction model.

SS1304: Obtain, through prediction based on a part and/or all of a first intervention plan of the user, a current value of a health indicator of the user, and the first individual health benefit prediction model, a predicted value of the health indicator after the part and/or all of the first intervention plan is executed.

After a personalized to-be-executed intervention plan of the user is generated in step S403, for example, after generating the first intervention plan, the electronic device may input the part and/or all of the first intervention plan and the current value of the health indicator of the user into the first individual health benefit prediction model, to obtain the predicted value of the health indicator obtained after the first intervention plan is executed.

A predicted value of the health indicator obtained after each cycle in the first intervention plan is executed may be specifically included.

The predicted value of the health indicator may be presented in different manners, for example, may be presented by using a change trend of the health indicator, or may be presented by using a numerical value of the health indicator. This is not limited herein.

By using the intervention plan benefit prediction method in this embodiment of this application, after making the first intervention plan, the electronic device may directly predict the predicted value of the health indicator obtained after the first intervention plan is executed, and may display the predicted value on the electronic device for the user to view. This enables the user to understand a possible effect obtained after the intervention plan is executed, so that the user has a stronger motivation to execute the intervention plan. This greatly improves confidence and subjective initiative of the user in executing the intervention plan, and provides a comparative input for a post-intervention effect assessment. In addition, a health benefit model is established from coarse to fine in a device-cloud collaboration manner, so that a more accurate device-side model is obtained while user privacy and security are fully ensured.

It should be noted that the process of training the first individual health benefit prediction model is merely an example in this application. In actual application, there may be another manner of obtaining the first individual health benefit prediction model through training. For example, the first individual health benefit prediction model may be obtained through model training by using the machine learning algorithm and directly using individual training data of a first user, and the like. This is not limited herein.

(4) Intervention execution phase

S405: The electronic device delivers the intervention plan to another device in the health management system.

Optionally, the electronic device may directly send the first intervention plan generated in step S403 to each other device in the health management system, and each device determines, based on a capability of the device, which types of plans in the first intervention plan are to be monitored.

Optionally, the electronic device may send, to a corresponding device based on a capability of each device in the health management system, a plan that is in the first intervention plan and that matches the capability of each device. This is not limited herein.

For example, the another device in the health management system may include an intelligent wearable device, and/or a health check device, and/or an intelligent fitness device.

The electronic device may deliver a wearable intervention sub-plan in the first intervention plan to the intelligent wearable device. The wearable intervention sub-plan is a part or all of the first intervention plan. The wearable intervention sub-plan includes a part or all of the first exercise plan, and/or a part or all of the first diet plan, and/or a part or all of the first health habit check-in task set. The wearable intervention sub-plan is a plan that is in the first intervention plan and that is to be executed by the intelligent wearable device.

The electronic device delivers a check intervention sub-plan in the first intervention plan to the health check device. The check intervention sub-plan is a part or all of the first intervention plan. The check intervention sub-plan includes a part or all of health indicator check tasks in the first health habit check-in task set. The check intervention sub-plan is a plan that is in the first intervention plan and that is to be executed by the health check device.

The electronic device delivers a fitness intervention sub-plan in the first intervention plan to the intelligent fitness device. The fitness intervention sub-plan is a part or all of the first intervention plan. The fitness intervention sub-plan includes a part or all of the first exercise plan. The fitness intervention sub-plan is a plan that is in the first intervention plan and that is to be executed by the intelligent fitness device.

Optionally, the electronic device may directly send intervention plans of all cycles in the first intervention plan to each other device, or may first send intervention plans of a part of cycles in the first intervention plan to each other device (for example, may send only an intervention plan of a first cycle in the first intervention plan to each other device). This is not limited herein.

For example, FIG. 14 is a schematic diagram of an example of delivering intervention plans of different types to different devices according to an embodiment of this application. If the first intervention plan is generated in step S403, the first intervention plan includes the plans of three types: the exercise plan 101, the diet plan 102, and the check-in task 103.

The electronic device may send the part or all of the first exercise plan in the first intervention plan to the intelligent fitness device in the health management system, so that the intelligent fitness device performs exercise configuration based on the first exercise plan, and records actual execution data of executing the first exercise plan by the user.

The electronic device may send the part or all of health indicator check tasks in the first health habit check-in task set in the first intervention plan to the health check device in the health management system, so that the health check device reminds, based on the health indicator check task, the user to perform health check, and records measured health data of the user.

The electronic device may send a part or all of the first exercise plan, the first diet plan, and the first health habit check-in task set in the first intervention plan to the intelligent wearable device in the health management system, so that the intelligent wearable device reminds the user to execute the first intervention plan, records actual execution data of the intervention plan, and monitors health data of the user during execution of the intervention plan.

It may be understood that, after the intervention solution is generated, there may be no specific execution sequence for (3) Benefit prediction phase and (4) Intervention execution phase. In some embodiments, (3) Benefit prediction phase may be executed first, and then (4) Intervention execution phase is executed. In some embodiments, (4) Intervention execution phase may be executed first, and then (3) Benefit prediction phase is executed. In some embodiments, (3) Benefit prediction phase and (4) Intervention execution phase may be simultaneously executed. This is not limited herein.

(5) Data monitoring phase

S406: The intelligent wearable device monitors the actual execution data of the intervention plan and the health data.

For example, after the electronic device sends the first intervention plan to the intelligent wearable device, the intelligent wearable device may monitor the actual execution data of the first intervention plan and the health data.

Specifically, for the first exercise plan in the first intervention plan, the intelligent wearable device may remind the user to exercise, and monitor the actual execution data during exercise. If the intelligent wearable device has a health check module, values of the health indicator before and after the user executes the intervention plan may be further obtained.

Specifically, for the first diet plan in the first intervention plan, the intelligent wearable device may remind the user to eat according to the first diet plan.

Specifically, for the first health habit check-in task set in the first intervention plan, the intelligent wearable device may remind the user to complete check-in task content. For a check-in task whose completion status can be monitored by the intelligent wearable device, the intelligent wearable device may automatically complete the check-in task after detecting that the user completes the task. This is not limited herein.

S407: The health check device measures the health data.

For example, after the electronic device sends the health indicator check task in the first intervention plan to the health check device, the health check device may remind, based on the health indicator check task, the user to measure the health indicator, and record a value of the health indicator measured each time.

S408: The intelligent fitness device monitors the actual execution data of the intervention plan.

For example, after the electronic device sends the first exercise plan in the first intervention plan to the intelligent fitness device, the intelligent fitness device may perform exercise configuration each time based on the first exercise plan. For example, the intelligent fitness device may configure an exercise type, exercise frequency, exercise intensity, exercise time, and the like of each exercise based on the first exercise plan, and may monitor an actual execution status of executing the first exercise plan. The intelligent fitness device may further periodically remind, based on the first exercise plan, the user to start exercise.

S409: The electronic device monitors the actual execution data of the intervention plan and the health data.

For example, the electronic device may alternatively monitor an actual execution status of the first intervention plan and a value of the health indicator.

Specifically, for the first exercise plan in the first intervention plan, the electronic device may remind the user to exercise, and monitor the actual execution data during exercise. If the electronic device has a health check module, values of the health indicator before and after the user executes the intervention plan may be further obtained.

Specifically, for the first diet plan in the first intervention plan, the electronic device may remind the user to eat according to the first diet plan, and record a diet status.

Specifically, for the first health habit check-in task set in the first intervention plan, the electronic device may remind the user to complete check-in task content. For a check-in task whose completion status can be monitored by the electronic device, the electronic device may automatically complete the check-in task after detecting that the user completes the task. For a check-in task whose completion status cannot be monitored by the electronic device, the electronic device may accept an operation of the user to complete the check-in task.

S410: The intelligent wearable device, the health check device, and the intelligent fitness device all send, to the electronic device, actual execution data and health data that are monitored or detected.

In an execution process of the first intervention plan, each device in the health management system can send the actual execution status and the value of the health indicator that are monitored to the electronic device.

After determining that execution of one cycle in the first intervention plan is completed, and obtaining an actual execution status and a value of the health indicator of the one cycle in the first intervention plan, the electronic device may perform (6) the effect assessment and intervention plan adjustment phase.

(6) Effect assessment and intervention plan adjustment phase

S411: The electronic device runs, based on the actual execution data and the health data that are collected after one cycle in the intervention plan is executed, an effect assessment model to assess an effect of an actual execution status of an intervention plan of a current cycle, and provides a recommendation.

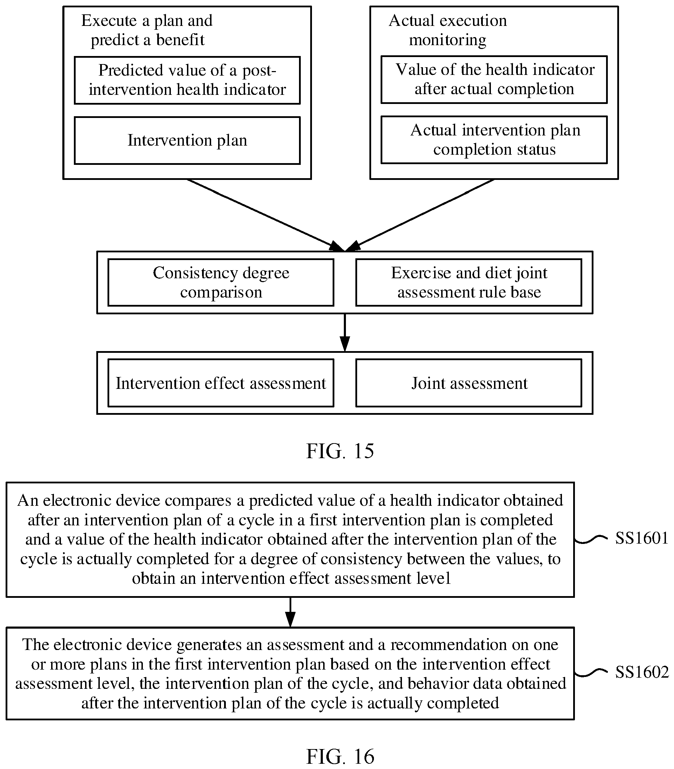

With reference to a schematic diagram of an example of an information flow direction in a process of generating an intervention plan effect assessment shown in FIG. 15 and a schematic flowchart of an intervention plan effect assessment shown in FIG. 16, the following describes an intervention plan effect assessment method according to an embodiment of this application.

SS1601: An electronic device compares a predicted value of a health indicator obtained after an intervention plan of a cycle in a first intervention plan is completed and an actual value of the health indicator obtained after the intervention plan of the cycle is completed for a degree of consistency between the values, to obtain an intervention effect assessment level.

In some embodiments of this application, the intervention effect assessment level is an intervention effect assessment result. The intervention effect assessment result may alternatively be represented in another form other than a level, for example, a score or a percentage. This is not limited herein. Based on step S404, after generating the first intervention plan, the electronic device may obtain a predicted value of the health indicator after an intervention plan of each cycle in the first intervention plan is completed.

Based on step S410, after an intervention plan of one cycle in the first intervention plan is executed, the electronic device may obtain a value of the health indicator before the intervention plan of the cycle is executed, and a value of the health indicator after the intervention plan of the cycle is actually completed.

For example, after an intervention plan of a first cycle in the first intervention plan is executed, the electronic device may compare a predicted value of the health indicator (for example, a body weight, BMI, a body fat percentage, systolic blood pressure, diastolic blood pressure, fasting plasma glucose, total cholesterol, or triglyceride) obtained after the intervention plan of the first cycle is completed and an actual value of the health indicator obtained after the intervention plan of the first cycle is completed for a degree of consistency between the values, to obtain the intervention effect assessment level.

A specific intervention effect assessment level may be expressed in various manners, for example, by star rating, 100-point system score, or numerical rating. Different levels may be divided in various preset manners. This is not limited herein.

For example, one representation and division manner of the intervention effect assessment level is used for description in the following.

Intervention effect assessment level can be divided into five levels: S, A, B, C, and D.

A trigger condition of S is that an actual health indicator reaches or is better than a predicted health indicator.

A trigger condition of A is that an actual health indicator is better than a health indicator before the intervention plan is executed, but an improved change value is within 30% less than a predicted value. This may be understood as follows: (Actual health indicator - Health indicator before the intervention plan is executed) < (Predicted health indicator - Health indicator before the intervention plan is executed) $\times$ (1 - 30%).

A trigger condition ofB is that an actual health indicator is better than a health indicator before the intervention plan is executed, but an improved change value is within 60% less than a predicted value. This may be understood as follows: (Actual health indicator - Health indicator before the intervention plan is executed) < (Predicted health indicator - Health indicator before the intervention plan is executed) $\times$ (1 - 60%).

A condition of C is that an actual health indicator is better than a health indicator before the intervention plan is executed, but a benefit effect does not reach that of B or above.

A condition of D is that an actual health indicator is worse than a health indicator before the intervention plan is executed.

SS1602: The electronic device generates an assessment and a recommendation on one or more plans in the first intervention plan based on the intervention effect assessment level, the intervention plan of the cycle, and behavior data obtained after the intervention plan of the cycle is actually completed.

After obtaining the intervention effect assessment level of the cycle in the first intervention plan, the electronic device may generate the assessment and the recommendation on the one or more plans in the first intervention plan based on a preset assessment rule base, the intervention plan of the cycle, and the behavior data obtained after the intervention plan of the cycle is actually completed.

Specifically, positive and negative assessments and recommendations may be provided for the one or more plans based on the intervention effect assessment level obtained in SS1602, the behavior data obtained after the intervention plan of the cycle is actually completed, and the intervention plan, and a factor that a user does not complete according to the plan is collected. In addition, assessments and recommendations on items related to exercise and diet are generated based on an exercise and diet joint assessment rule base based on a diet and exercise balance theory. According to the intervention effect assessment method in this embodiment of this application, an actual effect of completing the intervention plan can be assessed by comparing an inference result of a health benefit prediction model with an actual change of the health indicator and based on the exercise and diet joint assessment rule base. This resolves a problem that closed-loop health management cannot be implemented. This implements closed-loop health management, improves user experience, and achieves long-term health promotion.

S412: The electronic device adjusts the intervention plan of the next cycle based on the assessment result.

Specifically, the electronic device may adjust the intervention plan of the next cycle in the first intervention plan based on an assessment and a recommendation on each plan in the first intervention plan and a reason that is entered by the user and for which the intervention plan of the cycle is not completed, to ensure that the intervention plan can be implemented and an optimal effect is achieved.

It may be understood that the intervention plan of the next cycle may be adjusted in various manners, and there may be various preset adjustment rules. Principles of these preset adjustment rules are as follows: 1. The user can execute the rules. 2. Maximize benefits.

For example, the following uses several adjustment rules for description.

Adjustment policy 1: The user gives an assessment on subjective experience after completing exercise (exercise training load is assessed based on subjective experience, and selected from a range of 1 to 10, where 1 is very easy and 10 is very exhausting), R indicates a result selected by the user. A heart rate range of the next cycle is adjusted as follows: Heart rate range set at a current phase + 2 × (R - 5). If a quantity of times for exercise per week is less than or equal to 4 and R ≥ 8, a quantity of times for exercise per week in the next cycle is adjusted to 5, exercise duration of each time is reduced, but total weekly exercise duration remains unchanged.

Adjustment policy 2: If the assessment result is that fat burning of the user enters the plateau (even if the user completes the set intervention plan, the body weight does not lose and even rebounds for more than two consecutive weeks), a resistance exercise session is added to the existing aerobic exercise intervention, and a high intensity interval training (HIIT) plan is added for a low-risk exercise user with a high cardiorespiratory endurance level based on the cardiorespiratory endurance level of the user.

It may be understood that after the intervention plan of the next cycle is adjusted, the electronic device may perform steps S404 to S412 again based on an adjusted intervention plan.

In this embodiment of this application, health benefits are predicted before the intervention plan is executed. This greatly improves subjective initiative and compliance of the user in executing the intervention plan. In addition, the predicted health indicator, the actual health indicator after the intervention plan is executed, and the user behavior data can be used to assess the generated intervention plan, so as to adjust the intervention plan, so that health management reaches a real closed-loop mode. This can effectively improve user health while improving user compliance, and implement a virtuous cycle process.

It may be understood that, in the foregoing embodiment, an example in which there is only one user of each device in the health management system is used for description. In actual application, if each device in the health management system has a plurality of users (for example, the electronic device has a plurality of users), the steps in the foregoing embodiment may be specific to different users, and steps S401 to S411 may be separately performed for each user. In this case, the collected data, the generated intervention plan, and the like are specific to only one user in the plurality of users, and related data of different users may be distinguished by different user identifiers. This is not limited herein.

In the foregoing embodiment, a current health status of the user may be reflected by recognizing a health risk factor of the user, and then an intervention plan that helps improve the health status of the user is generated. An embodiment of this application further provides a user age estimation method. A health risk level of a user may be comprehensively assessed by using an estimated user age, so that a health status of the user can be managed more accurately.

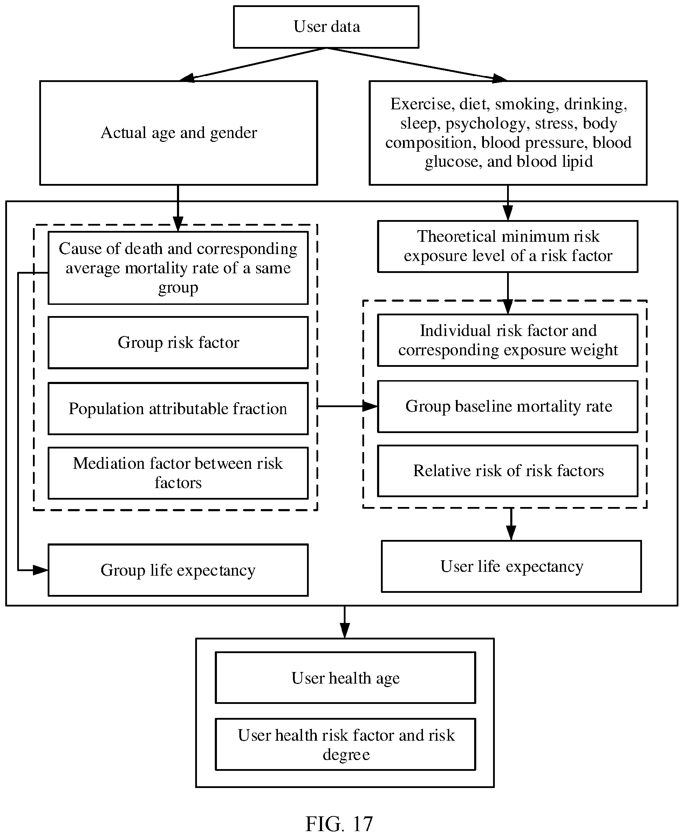

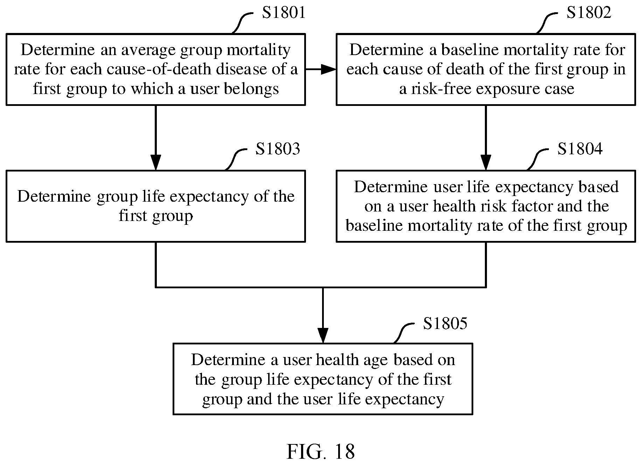

With reference to a schematic diagram of an example of an information flow direction in a user age estimation process shown in FIG. 17 and a schematic flowchart a user age estimation method shown in FIG. 18, the following describes a user age estimation method according to an embodiment of this application.

Data preparations that may be performed first are as follows.

Data preparation (1): A national population cause of death and population data over the years can be obtained based on cause of death and risk factor monitoring data in a burden of disease database (for example, the GBD database), including:

normal cause of death and number of deaths: indicating a number of deaths for each cause of death;
population risk factor: indicating a set of average exposure risk factors in different groups; and denoted by J below;
population attributable fraction (Population Attributable Fraction, PAF): indicating a statistical indicator of an effect of an exposure risk factor on an occurrence of a disease in a population, indicating a proportion of an incidence of a disease caused by a specific risk factor in a total incidence of the disease in the population, and also understood as a proportion of which the incidence of the disease in the population is reduced after the risk factor is eliminated, where a $PAF_{jo}$ appearing below indicates a PAF for a risk factor j and a cause-of-death disease o;
risk factor mediation factor (Mediation Factor, MF): indicating an effect of a first risk factor on a second risk factor on a causal effect path of a disease and an effect size, where an $MF_{ijo}$ appearing below indicates an MF of a risk factor i for the risk factor j and the cause-of-death disease o; and
risk factor relative risk (Relative Risk, RR): indicating a ratio of an incidence of a disease in a group exposed to a risk factor to that not exposed to the risk factor, where a greater relative risk indicates a greater exposure effect, that is, greater intensity of association between exposure and a result, and $RR_{jo}$ indicates an RR of the risk factor j for the cause-of-death disease o.

Further, the data may be divided into data of different groups. For example, a group division manner may be shown in Table 5.

**Table 5**

| Gender | Age | Group |
|---|---|---|
| Male | $\leq 18$ | Group 1 |
| Male | > 18 and $\leq 30$ | Group 2 |
| Female | $\leq 18$ | Group 3 |
| Female | > 18 and $\leq 30$ | Group 4 |
| ... | ... | ... |

Data preparation (2): An average group mortality rate (Mortality Rate, $MR_o$) for each cause-of-death disease (for example, a coronary heart disease, a cerebral stroke, diabetes, or a lung cancer) of each group can be calculated based on data of the group.

For example, if a total population of Group 2 is 100,000 and a number of deaths due to the coronary heart disease is 500, an average group mortality rate for the coronary heart disease of the group is 500/100000 = 0.5%.

Data preparation (3): Group life expectancy SE of each group can be calculated based on the average group mortality rate $MR_o$ for each cause-of-death disease of each group.

<1> A mortality rate $q_o$ for each cause-of-death disease in each subsequent year (up to 120 years of life expectancy) can be calculated based on the average group mortality rate $MR_o$.

For example, a manner of calculating the mortality rate $q_o$ may be shown in the following formula (1):

$$q_o = \frac{MR_o}{1 + 0.5 \times MR_o} \text{ Formula (1)}$$

Herein, o indicates a single cause-of-death disease.

<2> An all-cause mortality rate Q of the group in each subsequent year can be obtained based on the mortality rate $q_o$ for each cause-of-death disease.

For example, a manner of calculating the all-cause mortality rate Q may be shown in the following formula (2):

$$Q = 1 - \prod_{o}^{M} (1 - q_o) \text{ Formula (2)}$$

Herein, M indicates a set of cause-of-death diseases.

<3> The group life expectancy SE can be calculated by using a mathematical expectation method based on the all-cause mortality rate Q of the group in each subsequent year.

For example, a manner of calculating the group life expectancy SE may be as follows:
A value of a probability *i of the group not dying at an age i is calculated by using the following formula (3):

$$t_i = (1 - Q_a) \times (1 - Q_{a+1}) \times (1 - Q_{a+2}) \times \cdots \times (1 - Q_{i-1}) \times Q_i \times i \text{ Formula (3)}$$

Herein, a is an actual age of the user, i indicates a group lifespan (ranging from a to 120), and $t_i$ indicates a probability of the group living to the age i multiplied by the age i. Another expression of living to the age i is that the group does not die from the age a to an age i-1, and dies at the age i.

Then, the group life expectancy SE is obtained by accumulating $t_a$ to $t_{120}$ by using the following formula (4):

$$SE = t_a + t_{a+1} + t_{a+2} + \cdots + t_{120} \quad \text{Formula (4)}$$

Data preparation (4): A baseline mortality rate $BD_o$ for each cause-of-death disease of each group in a risk-free exposure case can be determined based on the average group mortality rate $MR_o$ for each cause of death for each group, the population attributable fraction $PAF_{jo}$ and the risk factor mediation factor $MF_{ijo}$, where the risk-free exposure case is a hypothetical case in which everyone in the group has no health risk factor.

For example, a manner of calculating the baseline mortality rate $BD_o$ for each cause of death of the group in the risk-free exposure case may be as follows:

The population attributable fraction $PAF_{Jo}$ of a set of exposure risk factors can be calculated according to the following formula (5):

$$PAF_{Jo} = 1 - \prod_{j=1}^{J}\left[1 - PAF_{jo} \times \prod_{i=1}^{I}(1 - MF_{ijo})\right] \quad \text{Formula (5)}$$

Herein, J is a set of risk factors for group exposure; and I is a set of risk factors related to an effect of the risk factor j on the cause-of-death disease o.

The baseline mortality rate $BD_o$ for each cause of death of the group in the risk-free exposure case is calculated according to the following formula (6):

$$BD_o = MR_o \times (1 - PAF_{Jo}) \quad \text{Formula (6)}$$

Data preparation (5): A mortality rate $q_o^b$ for each cause of death in each group in the risk-free exposure case in each subsequent year (up to 120 years of life expectancy) can be determined based on the baseline mortality rate $BD_o$ for each cause of death of each group in the risk-free exposure case.

For example, a manner of calculating the mortality rate $q_o^b$ for each cause of death in each subsequent year may be shown in the following formula (7):

$$q_o^b = \frac{BD_o}{1 + 0.5 \times BD_o} \quad \text{Formula (7)}$$

It may be understood that, the foregoing data preparation may be completed on the cloud, or may be completed after the electronic device obtains a related device, or may be completed completely, or may be completed partially. This is not limited herein.

After some data is prepared, the following steps may be performed.

S1801: Determine an average group mortality rate for each cause-of-death disease of a first group to which a user belongs.

Specifically, the first group to which the user belongs may be first determined based on collected basic user information, and then the average group mortality rate for each cause-of-death disease of the first group is obtained.

For example, if the basic user information indicates that a gender is male and an age is 25 years old, and a group division manner shown in Table 5 is used, it may be determined that the user belongs to Group 2: 18 to 30 years old, and male. Then, an average group mortality rate for each cause-of-death disease corresponding to Group 2 is obtained.

If a cloud has prepared in advance an average group mortality rate for each cause-of-death disease of each group, an electronic device may directly obtain the average group mortality rate for each cause-of-death disease of Group 2 from the cloud.

If a cloud has not prepared in advance an average group mortality rate for each cause-of-death disease of each group, an electronic device may obtain required data and then calculate the average group mortality rate for each cause-of-death disease of Group 2 according to the calculation manner in Data preparation (3).

S1802: Determine a baseline mortality rate for each cause-of-death disease of the first group in a risk-free exposure case.

If the cloud has prepared in advance a baseline mortality rate for each cause-of-death disease of each group in the risk-free exposure case, the electronic device may directly obtain, from the cloud, the baseline mortality rate for each cause-of-death disease of the first group in the risk-free exposure case.

If the cloud has not prepared in advance the average group mortality rate for each cause-of-death disease of each group, the electronic device may obtain required data and then calculate the baseline mortality rate for each cause-of-death disease of the first group in the risk-free exposure case according to the calculation manner in Data preparation (4).

S1803: Determine group life expectancy of the first group.

If the cloud has prepared in advance group life expectancy of each group, the electronic device may directly obtain the group life expectancy of the first group from the cloud.

If the cloud has not prepared in advance group life expectancy of each group, the electronic device may obtain required data and then calculate the group life expectancy of the first group according to the calculation manner in Data preparation (3).

S1804: Determine user life expectancy based on a user health risk factor and the baseline mortality rate of the first group.

It may be understood that, because user health risk factors are different, values or exposure levels of the risk factors in the user health risk factors are different, different user life expectancy may be determined.

For example, a calculation process may be shown as follows:

<1> A relative risk degree of an overall user exposure risk factor set and each cause-of-death disease (which may be referred to as an overall user risk factor relative risk degree in this application) $RR_{Ko}$ is determined based on a recognized user health risk factor.

Optionally, the user health risk factor may be recognized in the manner in step S402. This is not limited herein.

For example, a manner of calculating the overall user risk factor relative risk degree $RR_{Ko}$ may be shown in the following formula (8):

$$RR_{Ko} = \prod_{k=1}^{K}\left[\left(1 + (RR_{ko} - 1) \times \prod_{l=1}^{L}(1 - MF_{ijo})\right)^{m_k}\right] \quad \text{Formula (8)}$$

Herein, K is a set of risk factors in the user health risk factor; L is a set of risk factors related to an effect of a risk factor k on the cause-of-death disease o; and $m_k$ is an exposure weight of an exposure level of the risk factor k in the user relative to a theoretical minimum risk exposure level (Theoretical minimum risk exposure level, TMREL) of the risk factor.

Optionally, a relative risk degree ($RR_{kM}$) corresponding to each risk factor in the user health risk factor and all cause-of-death diseases may be further calculated according to a formula (9). The user exposure risk factors are ranked in descending order of $RR_{kM}$.

$$RR_{kM} = \prod_{o}^{M}\left[\left(1 + (RR_{ko} - 1) \times \prod_{l=1}^{L}(1 - MF_{ijo})\right)^{mk}\right] \quad \text{Formula (9)}$$

<2> A mortality rate $q'_0$ for each cause-of-death disease of the user in each subsequent year (up to 120 years of life expectancy) can be calculated based on the overall user risk factor relative risk degree $RR_{Ko}$.

For example, a manner of calculating the mortality rate $q'_o$ for each cause-of-death disease of the user in each subsequent year based on the overall user risk factor relative risk degree $RR_{Ko}$ may be shown in the following formula (10):

$$q'_o = RR_{Ko} \times q_o^b \text{ Formula (10)}$$

<3> An all-cause mortality rate Q' of the user in each subsequent year can be obtained based on the mortality rate $q'_o$ for each cause-of-death disease of the user.

For example, a manner of calculating the all-cause mortality rate Q' of the user in each subsequent year based on the mortality rate $q'_o$ for each cause-of-death disease of the user may be shown in the following formula (11):

$$Q' = 1 - \prod_o^M (1 - q'_o) \text{ Formula (11)}$$

Herein, M indicates a set of causes of death.

<4> The user life expectancy AE can be calculated by using a mathematical expectation method based on the all-cause mortality rate *Q'* of the user in each year.

For example, a manner of calculating the user life expectancy AE may be shown in the following formula (12) and formula (13):

$$t'_i = (1 - Q'_a) \times (1 - Q'_{a+1}) \times (1 - Q_{a+2}) \times \cdots \times (1 - Q'_{i-1}) \times Q'_i \times i \text{ Formula (12)}$$

Herein, a is an actual age of the user, i indicates a user lifespan (ranging from a to 120), and $t'_i$ indicates a probability of the user living to the age i multiplied by the age i. Another expression of living to the age i is that the user does not die from the age a to an age i-1, and dies at the age i. In the formula (12), $1 - Q'_{i-1}$ indicates a probability that the user does not die at the age i-1, and $Q'_i$ indicates a probability that the user dies at the age i.

As shown in the following formula (13), the user life expectancy AE is obtained by accumulating $t'_a$ to $t'_{120}$:

$$AE = t'_a + t'_{a+1} + t'_{a+2} + \cdots + t'_{120} \text{ Formula (13)}$$

S1805: Determine a user health age based on the group life expectancy of the first group and the user life expectancy.
For example, a calculation manner may be: adding the actual age a of the user to the life expectancy of the first group, and then subtracting the user life expectancy, to obtain the user health age.
For example, if the actual age of the user is 25 years old, the calculated group life expectancy of the first group is 70 years old, and the calculated user life expectancy is 60 years old, the user health age is 25 + 70 - 60 = 35 years old.
In this embodiment of this application, the user health age is determined, so that a measurable risk level can be provided for an overall user health risk while a user exposure risk factor is recognized. In addition, a ranking of user health risk factors can be established, to provide a basis for a priority of an intervention policy in health management.
In some embodiments of this application, the user health age may also be referred to as an estimated user age.
The foregoing user health age assessment method may be applied to each phase of the health management method in this application.
In (1) Assessment phase of the health management method shown in FIG. 4A and FIG. 4B, the electronic device may recognize the user health risk factor and the user health age based on the collected user data.

In (2) Intervention plan generation phase of the health management method shown in FIG. 4 A and FIG. 4B, the electronic device may generate personalized intervention plans of a plurality of cycles based on the user health risk factors and the ranking of the risk factors.

In (3) Benefit prediction phase of the health management method shown in FIG. 4A and FIG. 4B, data input into a benefit health model by the electronic device may include the user health age recognized in (1) Assessment phase, and a predicted health indicator may include the user health age obtained after the intervention plan is executed.

It may be understood that, after the intervention plan is executed, a value of each user health risk factor changes. Therefore, the user health age calculated based on the user health risk factor also changes accordingly.

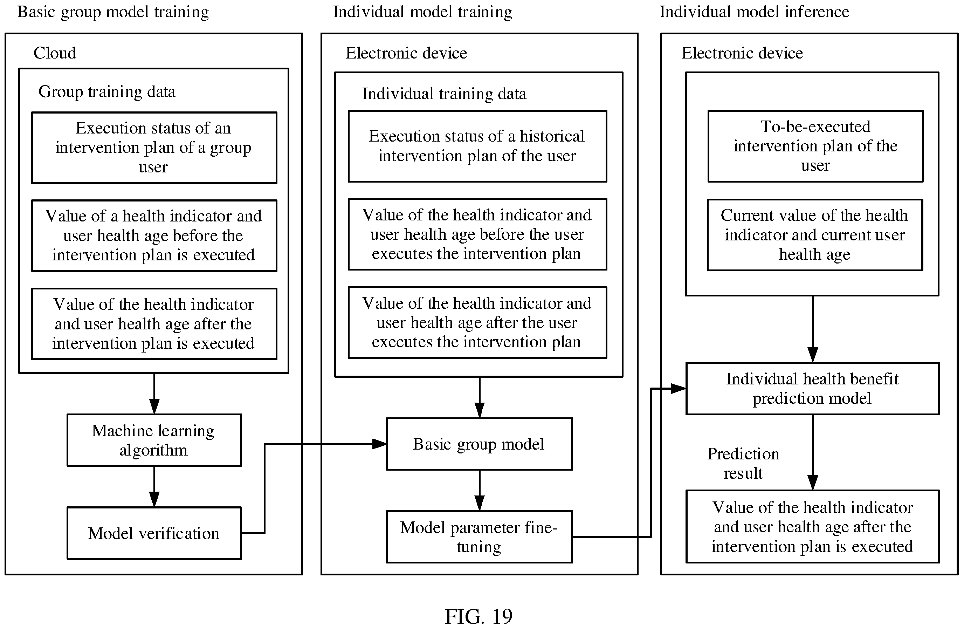

The following specifically describes another intervention plan benefit prediction method in an embodiment of this application with reference to a schematic diagram of an information flow direction shown in FIG. 19.

In a process of training a benefit prediction model:

A process of establishing a basic group model on a cloud may be as follows:

<1> With knowledge and consent of a user, user data, an intervention plan, an intervention plan execution status, a value of a health indicator before the intervention plan is executed, a user health age before the intervention plan is executed, a value of the health indicator after the intervention plan is executed, and a user health age after the intervention plan is executed that are on an electronic device are anonymized and then uploaded to the cloud.

<2> A large amount of data uploaded to the cloud by mobile electronic devices belonging to different users is sorted and cleaned, and is divided into group training data for different groups based on age groups and genders.

<3> FIG. 12 is a schematic diagram of an example of a training process of a basic group model.

Group training data for a group can be divided into a training data set and a validation data set, and the basic group model can be supervised by using a machine learning neural network or a regression algorithm. An independent variable is a weekly intervention plan execution status (average daily exercise duration, an exercise heart rate, a dietary calorie intake, three nutrient intakes, sleep duration, an amount of smoking and an amount of drinking, or the like) of a user in the group, a value of a health indicator (a body weight, BMI, a body fat percentage, systolic blood pressure, diastolic blood pressure, fasting plasma glucose, total cholesterol, triglyceride, or the like) before an intervention plan is executed, and the user health age before the intervention plan is executed. A dependent variable is a value of the health indicator after the intervention plan is executed, and the user health age after the intervention plan is executed. An objective function of the machine learning neural network or the regression algorithm may be a mean square error of the user health ages before and after the intervention plan is executed.

The objective function may be optimized by using actual values of the user health indicator and a user health age that are obtained after the intervention plan in the training data set is executed, so that differences between a predicted value of the user health indicator and the user health age that are obtained by using the machine learning neural network or the regression algorithm after the intervention plan is executed and the actual values of the user health indicator and the user health age that are obtained after the intervention plan is executed are within a preset range, to obtain a to-be-verified basic group model.

Then, data in the validation data set may be used to verify the to-be-verified basic group model. If the differences between the predicted values of the user health indicator and the user health age that are obtained after the intervention plan is executed and the actual values of the user health indicator and the user health age that are obtained after the intervention plan is executed are within the preset range, it can be determined that the basic group model of the group is obtained.

It may be understood that, for different groups, different basic group models may be obtained through training. This is not limited herein.

A process of establishing an individual health benefit prediction model on the electronic device based on the basic group model may be as follows:

<1> A basic group model of a first group matching basic user information of the electronic device is obtained from the cloud.

<2> An execution status of a historical intervention plan of the user, the value of the health indicator before the user executes the intervention plan, the user health age before the user executes the intervention plan, the value of the health indicator after the user executes the intervention plan, and the user health age after the user executes the intervention plan that are in the electronic device are used as individual training data of the user, to train the obtained basic group model of the first group, and fine-tune parameters of the basic group model, so that the individual health benefit prediction model of the user can be obtained.

With reference to the foregoing process of establishing the basic group model and the individual health benefit prediction model, the following describes in detail an intervention plan benefit prediction method according to an embodiment of this application.

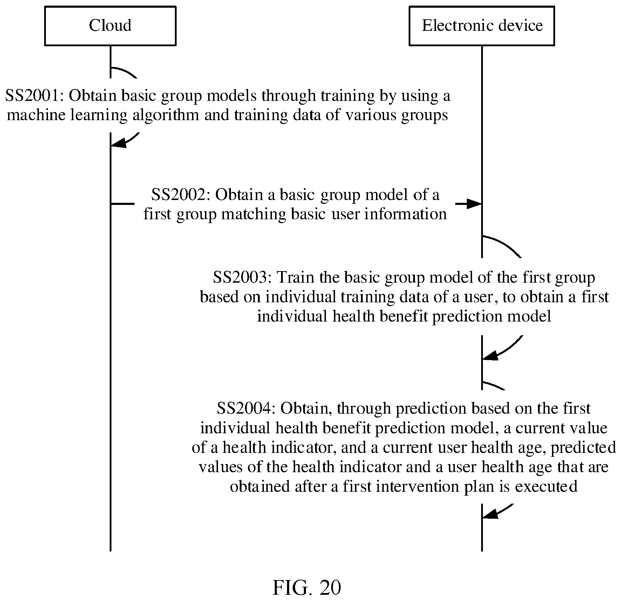

FIG. 20 is another schematic flowchart of an intervention plan benefit prediction method according to an embodiment of this application.

SS2001: Obtain basic group models through training by using a machine learning algorithm and training data of various groups.

It may be understood that, as described in the foregoing training process of the basic group model, each group training data may include a user health age obtained before an intervention plan is executed and a user health age obtained after the intervention plan is executed. Therefore, an input of each trained basic group model may include the user health age obtained before the intervention plan is executed, and an output may include the user health age obtained after the intervention plan is executed.

Other processes are similar to those in step SS1301. Details are not described herein again. SS2002: Obtain a basic group model of a first group matching basic user information.

This step is similar to step SS1302. Details are not described herein again.

SS2003: Train the basic group model of the first group based on individual training data of a user in an electronic device, to obtain a first individual health benefit prediction model.

It may be understood that, as described in the foregoing training process of the individual health benefit prediction model, the individual training data of the user may include the user health age obtained before the intervention plan is executed and the user health age obtained after the intervention plan is executed. Therefore, an input of the trained first individual health benefit prediction model may include the user health age obtained before the intervention plan is executed, and an output may include the user health age obtained after the intervention plan is executed.

Other processes are similar to those in step SS1303. Details are not described herein again.

SS2004: Obtain, through prediction based on the first individual health benefit prediction model, a current value of a health indicator of the user, and a current user health age, predicted values of the health indicator and a user health age that are obtained after a first intervention plan is executed. After a personalized to-be-executed intervention plan of the user is generated in step S403, for example, after generating the first intervention plan, the electronic device may input the first intervention plan, the current value of the health indicator of the user, and the current user health age into the first individual health benefit prediction model, to obtain the predicted values of the health indicator and the user health age that are obtained after the first intervention plan is executed. Other processes are similar to those in step SS1304. Details are not described herein again.

In this embodiment of this application, the parameter of the user health age is added to the benefit prediction model. Because the user health age can reflect an overall health status of the user, prediction of an execution effect of the intervention plan can be more accurate.

In (6) Effect assessment and intervention plan adjustment phase of the health management method shown in FIG. 4A and FIG. 4B,

when assessing an effect of an intervention plan, an electronic device may not only compare a predicted value of a health indicator obtained after an intervention plan of a cycle is completed and an actual value of the health indicator obtained after the intervention plan of the cycle is completed for a degree of consistency between the values, but also compare a predicted value of a user health age obtained after the intervention plan of the cycle is completed and an actual value of the user health age obtained after the intervention plan of the cycle is completed for a degree of consistency between the values, to obtain an effect assessment result of the intervention plan.

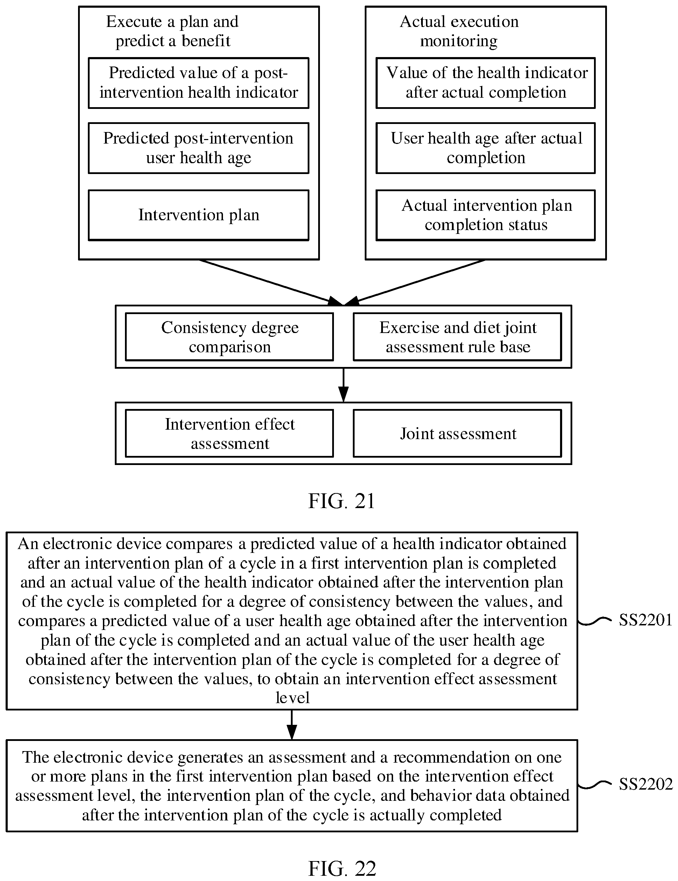

With reference to a schematic diagram of an information flow direction of an intervention plan effect assessment shown in FIG. 21 and a schematic flowchart of an intervention plan effect assessment method shown in FIG. 22, the following describes another intervention plan effect assessment method according to an embodiment of this application.

SS2201: An electronic device compares a predicted value of a health indicator obtained after an intervention plan of a cycle in a first intervention plan is completed and an actual value of the health indicator obtained after the intervention plan of the cycle is completed for a degree of consistency between the values, and compares a predicted value of a user health age obtained after the intervention plan of the cycle is completed and an actual value of the user health age obtained after the intervention plan of the cycle is completed for a degree of consistency between the values, to obtain an intervention effect assessment level.

It may be understood that, the intervention effect assessment level may be determined based on the degree of consistency between the health indicators and the degree of consistency between the user health ages in a plurality of specific manners. For example, it may be set that different health indicator consistency degrees correspond to different intervention effect assessment levels, different user health ages also correspond to different intervention assessment levels, and a lower intervention assessment level is selected as a final intervention effect assessment level. For example, if an intervention effect assessment level determined based on the health indicator consistency degree is B, and an intervention effect assessment level determined based on the user health age consistency degree is C, it may be determined that the final intervention effect assessment level is C. For another example, different intervention effect assessment levels may separately correspond to a health indicator consistency degree and a user health age consistency degree. Only when the health indicator consistency degree and the user health age consistency degree are met at the same time, a corresponding intervention effect assessment level may be determined. Alternatively, there may be many other determining manners, which are not limited herein.

Other processes are similar to those in step SS1601. Details are not described herein again.

SS2202: The electronic device generates an assessment and a recommendation on one or more plans in the first intervention plan based on the intervention effect assessment level, the intervention plan of the cycle, and behavior data obtained after the intervention plan of the cycle is actually completed.

This step is similar to step SS1602. Details are not described herein again.

According to the intervention effect assessment method in this embodiment of this application, the user health age is used in an assessment process. Because the user health age can reflect an overall health status of a user, an assessment result of the intervention plan is more accurate.

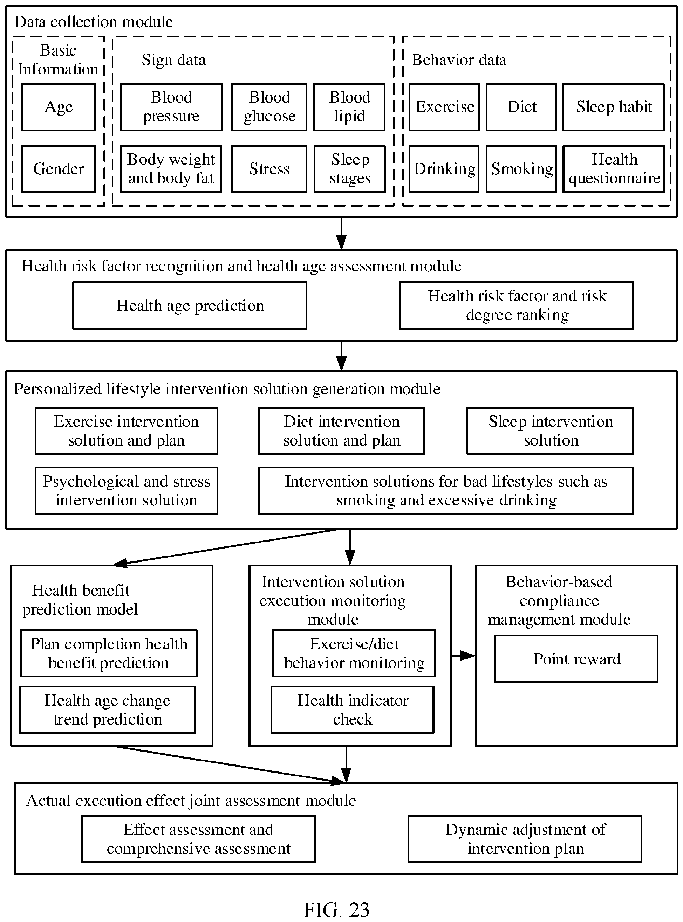

With reference to the foregoing steps and a schematic diagram of a software module architecture shown in FIG. 23, the following describes an example software system composition architecture of a health management system according to an embodiment of this application.

The health management system may include: a data collection module, a health risk factor recognition and health age assessment module, a personalized lifestyle intervention solution generation module, a health benefit prediction module, an intervention solution execution monitoring module, a behavior-based compliance management module, and an actual execution effect joint assessment module.

The data collection module may collect user data, including basic user information, for example, an age and a gender; user health data, for example, blood pressure, blood glucose, blood lipid, body composition, stress, and a sleep status; user behavior data, for example, exercise, diet, a sleep habit, drinking, smoking, and health questionnaire data; and the like. The data collection module may perform step S401.

The health risk factor recognition and health age assessment module may recognize a user health risk factor, calculate a user health age, and sort user health risk factors by risk degrees. The health risk factor recognition and health age assessment module may perform step S402 and the user health age assessment method in the embodiment of this application shown in FIG. 18.

The personalized lifestyle intervention solution generation module may generate various intervention plans beneficial to user health, such as exercise intervention solutions and plans, diet intervention solutions and plans, sleep intervention solutions, psychological and stress intervention solutions, and intervention solutions for bad lifestyles such as smoking and excessive drinking. The personalized lifestyle intervention solution generation module may perform step S403.

The health benefit prediction module may predict a health indicator obtained after an intervention plan is completed, and a user health age obtained after the intervention plan is completed. The health benefit prediction module may perform the intervention plan benefit prediction method according to embodiments of this application shown in FIG. 13 and FIG. 22.

The intervention solution execution monitoring module may detect behaviors such as exercise and diet, and related health indicators obtained before, after, and when the intervention plan is executed, to obtain actual execution data and health indicators of the intervention plan. The intervention solution execution monitoring module may perform steps S406 to S410.

The behavior-based compliance management module may reward the user with points after detecting that the user executes the intervention plan on time and by quantity.

The actual execution effect joint assessment module may assess an execution effect of the intervention plan, make a comprehensive assessment based on a diet status, and dynamically adjust the intervention plan based on an assessment result. The actual execution effect joint assessment module may perform steps S411 and S412 and the intervention plan effect assessment method according to this embodiment of this application.

The following describes an example of an electronic device 100 in a health management system according to an embodiment of this application.

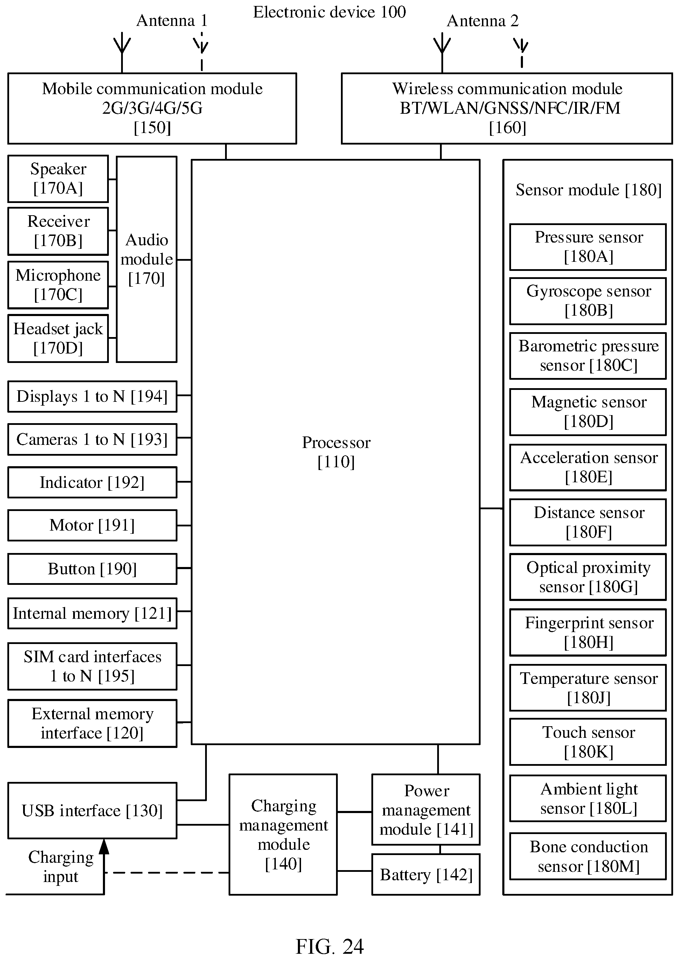

FIG. 24 is a schematic diagram of a structure of the electronic device 100 according to an embodiment of this application.

The electronic device 100 is used as an example below to describe embodiments in detail. It should be understood that the electronic device 100 may have more or fewer components than those shown in the figure, or may combine two or more components, or may have different component configurations. Various components shown in the figure may be implemented in hardware including one or more signal processing and/or application-specific integrated circuits, software, or a combination of hardware and software.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like. It may be understood that the structure shown in this embodiment of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by using hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may be a nerve center and a command center of the electronic device 100. The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes one serial data line (serial data line, SDA) and one serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be used to perform audio communication, and sample, quantize, and code an analog signal. In some embodiments, the audio module 170 may be coupled to the wireless communication module 160 through a PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be used for audio communication. The UART interface is a universal serial data bus, and is used for asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral component like the display 194 or the camera 193. The MIPI interface includes a camera serial interface (camera serial interface, CSI), a display serial interface (display serial interface, DSI), and the like. In some embodiments, the processor 110 communicates with the camera 193 through the CSI, to implement an image shooting function of the electronic device 100. The processor 110 communicates with the display 194 through the DSI interface, to implement a display function of the electronic device 100.

The GPIO interface may be configured by software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The SIM interface may be configured to communicate with the SIM card interface 195, to implement a function of transmitting data to an SIM card or reading data in an SIM card.

The USB interface 130 is an interface that conforms to a USB standard specification, and may be specifically a mini USB interface, a micro USB interface, a USB type-C interface, or the like. The USB interface 130 may be configured to connect to a charger to charge the electronic device 100, or may be configured to transmit data between the electronic device 100 and a peripheral device, or may be configured to connect to a headset for playing an audio through the headset. The interface may be further configured to connect to another electronic device, for example, an AR device.

It may be understood that an interface connection relationship between the modules shown in this embodiment of this application is merely an example for description, and constitutes no limitation on the structure of the electronic device 100. In some other embodiments of this application, the electronic device 100 may alternatively use an interface connection manner different from that in the foregoing embodiment, or use a combination of a plurality of interface connection manners. The charging management module 140 is configured to receive a charging input from the charger. The charger may be a wireless charger or a wired charger.

The power management module 141 is configured to connect to the battery 142, the charging management module 140, and the processor 110. The power management module 141 receives an input of the battery 142 and/or the charging management module 140, to supply power to the processor 110, the internal memory 121, an external memory, the display 194, the camera 193, the wireless communication module 160, and the like.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that includes 2G/3G/4G/5G or the like and that is applied to the electronic device 100. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules of the mobile communication module 150 may be disposed in the processor 110. In some embodiments, at least some functional modules of the mobile communication module 150 may be disposed in a same component as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by using an audio device (which is not limited to the speaker 170A, the receiver 170B, or the like), or displays an image or a video by using the display 194. In some embodiments, the modem processor may be an independent component. In some other embodiments, the modem processor may be independent of the processor 110, and is disposed in a same component as the mobile communication module 150 or another functional module.

The wireless communication module 160 may provide a wireless communication solution that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like and that is applied to the electronic device 100. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device 100 implements a display function by using the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information. The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1.

The electronic device 100 may implement an image shooting function by using the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of an image shooting scenario. In some embodiments, the ISP may be disposed in the camera 193.

The camera 193 is configured to capture a static image or a video. An optical image of an object is generated through the lens, and is projected onto the photosensitive element. The photosensitive element may be a charge-coupled device (charge-coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transformation on frequency energy.

The video codec is configured to compress or decompress a digital video. The electronic device 100 may support one or more video codecs. In this way, the electronic device 100 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor, quickly processes input information by referring to a structure of a biological neural network, for example, by referring to a mode of transmission between human brain neurons, and may further continuously perform self-learning. Applications such as intelligent cognition of the electronic device 100 may be implemented through the NPU, for example, image recognition, facial recognition, speech recognition, and text understanding.

The internal memory 121 may include one or more random access memories (random access memories, RAMs) and one or more nonvolatile memories (nonvolatile memories, NVMs).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM is usually referred to as a DDR5 SDRAM), and the like.

The nonvolatile memory may include a magnetic disk storage device and a flash memory (flash memory).

The flash memory may be classified into an NOR flash, an NAND flash, a 3D NAND flash, and the like according to an operation principle; may be classified into a single-level cell (single-level cell, SLC), a multi-level cell (multi-level cell, MLC), a triple-level cell (triple-level cell, TLC), a quad-level cell (quad-level cell, QLC), and the like based on a quantity of electric potential levels of a cell; or may be classified into a universal flash storage (English: universal flash storage, UFS), an embedded multimedia card (embedded multimedia card, eMMC), and the like according to storage specifications.

The random access memory may be directly read and written by using the processor 110, and may be configured to store an executable program (for example, a machine instruction) of an operating system or another running program, and may be further configured to store data of a user, data of an application, and the like.

The nonvolatile memory may also store an executable program, data of a user, data of an application, and the like, which may be loaded into the random access memory in advance for directly reading and writing by the processor 110.

The external memory interface 120 may be configured to connect to an external nonvolatile memory, to extend a storage capability of the electronic device 100. The external nonvolatile memory communicates with the processor 110 through the external memory interface 120, to implement a data storage function. For example, files such as music and videos are stored in the external nonvolatile memory.

The electronic device 100 may implement an audio function, for example, music playing and recording, by using the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to code and decode an audio signal. In some embodiments, the audio module 170 may be disposed in the processor 110, or some functional modules of the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an audio electrical signal into a sound signal. When a call is answered or speech information is received through the electronic device 100, the receiver 170B may be put close to a human ear to listen to a voice. The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and recognize a sound source, so as to implement a directional recording function and the like.

The headset jack 170D is configured to connect to a wired headset. The headset jack 170D may be the USB interface 130, or may be a 3.5 mm open mobile terminal platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch position based on a detection signal of the pressure sensor 180A. In some embodiments, touch operations that are performed in a same touch position but have different touch operation intensity may correspond to different operation instructions. For example, when a touch operation whose touch operation intensity is less than a first pressure threshold is performed on an icon of Messages, an instruction for viewing an SMS message is performed. When a touch operation whose touch operation intensity is greater than or equal to the first pressure threshold is performed on the SMS message application icon, an instruction for creating an SMS message is performed.

The gyroscope sensor 180B may be configured to determine a motion posture of the electronic device 100. In some embodiments, an angular velocity of the electronic device 100 around three axes (namely, axes x, y, and z) may be determined by using the gyroscope sensor 180B. The gyroscope sensor 180B may be configured to implement image stabilization during image shooting. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a clamshell phone, the electronic device 100 may detect opening and closing of a flip cover based on the magnetic sensor 180D. Further, automatic unlocking of the flip cover or a like feature is set based on a detected opening or closing state of the leather case or a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor 180E may be further configured to recognize a posture of the electronic device, and is used in switching between a landscape mode and a portrait mode, a pedometer, or a like application.

The distance sensor 180F is configured to measure a distance. The electronic device 100 may measure the distance in an infrared manner or a laser manner. In some embodiments, in an image shooting scenario, the electronic device 100 may measure a distance through the distance sensor 180F to implement quick focusing.

The optical proximity sensor 180G may include, for example, a light emitting diode (LED) and an optical detector, for example, a photodiode. The light emitting diode may be an infrared light emitting diode. The electronic device 100 emits infrared light by using the light emitting diode. The electronic device 100 detects infrared reflected light from a nearby object through the photodiode. When sufficient reflected light is detected, the electronic device 100 may determine that there is an object near the electronic device 100. When insufficient reflected light is detected, the electronic device 100 may determine that there is no object near the electronic device 100. The electronic device 100 may detect, by using the optical proximity sensor 180G, that the user holds the electronic device 100 close to an ear for a call, to automatically turn off a screen for power saving. The optical proximity sensor 180G may also be used in a smart cover mode or a pocket mode to automatically perform screen unlocking or locking.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 lowers performance of a processor located near the temperature sensor 180J, to reduce power consumption for thermal protection. In some other embodiments, when the temperature is less than another threshold, the electronic device 100 heats the battery 142 to prevent the electronic device 100 from being shut down abnormally due to a low temperature. In some other embodiments, when the temperature is lower than still another threshold, the electronic device 100 boosts an output voltage of the battery 142 to avoid abnormal shutdown caused by a low temperature.

The touch sensor 180K is also referred to as a touch panel. The touch sensor 180K may be disposed in the display 194, and the touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of the touch event. A visual output related to the touch operation may be provided by using the display 194. In some other embodiments, the touch sensor 180K may alternatively be disposed on a surface of the electronic device 100 at a location different from that of the display 194.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playing) may correspond to different vibration feedback effects. The motor 191 may also correspond to different vibration feedback effects for touch operations performed on different areas of the display 194. Different application scenarios (for example, a time prompt, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may also be compatible with different types of SIM cards. The SIM card interface 195 may also be compatible with an external storage card. The electronic device 100 interacts with a network through the SIM card, to implement functions such as conversation and data communication.

The processor 110 may invoke the computer instructions stored in the internal memory 121, so that the electronic device 100 performs the health management method, the intervention plan benefit prediction method, the intervention plan effect assessment method, and the user health age assessment method in embodiments of this application.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application. According to the context, the term "when" used in the foregoing embodiments may be interpreted as "if", "after", "in response to determining", or "in response to detecting". Similarly, according to the context, the phrase "when it is determined that" or "if (a stated condition or event) is detected" may be interpreted as a meaning of "if it is determined that", "in response to determining", "when (a stated condition or event) is detected", or "in response to detecting (a stated condition or event)". All or some of the foregoing embodiments may be implemented by using software, hardware, firmware, or any combination thereof. When software is used for implementation, all or some of the embodiments may be implemented in a form of a computer program product. The computer program product includes one or more computer instructions. When the computer program instructions are loaded and executed on a computer, the procedure or functions according to embodiments of this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

Persons of ordinary skill in the art may understand that all or some of the processes of the methods in embodiments may be implemented by a computer program instructing related hardware. The program may be stored in a computer-readable storage medium. When the program is run, the processes of the methods in embodiments are performed. The foregoing storage medium includes any medium that can store program code, like a ROM, a random access memory RAM, a magnetic disk, or an optical disc.

**Claims**

1. A health management method, wherein the method comprises:

   obtaining, by an electronic device, a first intervention plan generated based on user data of a first user; and
   predicting, by the electronic device, a predicted value of a health indicator obtained after a part and/or all of the first intervention plan is completed.

2. The method according to claim 1, wherein the obtaining, by an electronic device, a first intervention plan generated based on user data of a first user specifically comprises:

> obtaining, by the electronic device, the user data of the first user;
> recognizing, by the electronic device, a user health risk factor based on the user data; and
> generating, by the electronic device, the first intervention plan for the user health risk factor.

3. The method according to claim 1 or 2, wherein the user data comprises basic user information, user behavior data, and/or user health data.

4. The method according to claim 3, wherein the basic user information comprises an age and/or a gender;

> the user behavior data comprises at least one of exercise data, stress data, sleep data, diet data, drinking data, and smoking data; and
> the user health data comprises at least one of body weight data, body composition data, blood pressure data, blood glucose data, and blood lipid data.

5. The method according to claim 2, wherein the user data comprises basic user information and further comprises user behavior data and/or user health data, and the basic user information comprises an age and/or a gender; and the recognizing, by the electronic device, a user health risk factor based on the user data specifically comprises:

> obtaining, by the electronic device from risk factor correspondences of a plurality of groups, a risk factor correspondence of a first group corresponding to the basic user information, wherein different groups correspond to different age ranges and/or genders, and the risk factor correspondence of the first group comprises a correspondence between one or more health risk factors and a corresponding preset condition of the one or more health risk factors, and comprises a correspondence between a first health risk factor and a first preset condition; and
> determining, by the electronic device, the user health risk factor based on the user behavior data and/or the user health data and with reference to the risk factor correspondence of the first group, wherein when the user behavior data and/or the user health data meet/meets the first preset condition, the user health risk factor comprises the first health risk factor.

6. The method according to any one of claims 1 to 5, wherein the predicting, by the electronic device, a predicted value of a health indicator obtained after a part and/or all of the first intervention plan is completed specifically comprises:

> performing, by the electronic device, model training based on individual training data of the first user, to obtain a first individual health benefit prediction model, wherein the individual training data of the first user comprises an execution status of a historical intervention plan of the first user, a value of the health indicator before the historical intervention plan of the first user is executed, and a value of the health indicator after a part and/or all of the historical intervention plan of the first user is executed; and
> inputting, by the electronic device, the user health data in the user data and the part and/or all of the first intervention plan into the first individual health benefit prediction model, to predict the predicted value of the health indicator obtained after the part and/or all of the first intervention plan is completed.

7. The method according to any one of claims 1 to 6, wherein the health indicator comprises at least one of a body weight, a body mass index, a body fat percentage, systolic blood pressure, diastolic blood pressure, fasting plasma glucose, total cholesterol, and triglyceride.

8. The method according to any one of claims 1 to 7, wherein the method further comprises: displaying, by the electronic device, the predicted value of the health indicator obtained after the part and/or all of the first intervention plan is completed.

9. The method according to claim 8, wherein the first intervention plan comprises intervention plans of N cycles, N is a positive integer greater than 1, and the predicted value of the health indicator obtained after the part and/or all of the first intervention plan is completed comprises predicted values of the health indicator obtained after a part of and/or all cycles in the intervention plans of N cycles in the first intervention plan are separately completed; and the displaying, by the electronic device, the predicted value of the health indicator obtained after the part and/or all of the first intervention plan is completed specifically comprises: displaying, by the electronic device, a change trend of the health indicator obtained after the part and/or all of the first intervention plan is completed, wherein the change trend of the health indicator comprises the predicted values of the health indicator obtained after the part of and/or all cycles in the intervention plans of N cycles in the first intervention plan are separately completed.

10. The method according to any one of claims 1 to 9, wherein a health management system in which the electronic device is located further comprises an intelligent wearable device, and/or a health check device, and/or an intelligent fitness device; and
the method further comprises:

delivering, by the electronic device, a wearable intervention sub-plan in the first intervention plan to the intelligent wearable device, wherein the wearable intervention sub-plan is a plan that is in the first intervention plan and that is to be executed by the intelligent wearable device;
delivering, by the electronic device, a check intervention sub-plan in the first intervention plan to the health check device, wherein the check intervention sub-plan is a plan that is in the first intervention plan and that is to be executed by the health check device; and
delivering, by the electronic device, a fitness intervention sub-plan in the first intervention plan to the intelligent fitness device, wherein the fitness intervention sub-plan is a plan that is in the first intervention plan and that is to be executed by the intelligent fitness device.

11. The method according to claim 10, wherein the first intervention plan comprises a first exercise plan, and/or a first diet plan, and/or a first health habit check-in task set;

the wearable intervention sub-plan comprises a part or all of the first exercise plan, and/or a part or all of the first diet plan, and/or a part or all of the first health habit check-in task set;
the check intervention sub-plan comprises a part or all of health indicator check tasks in the first health habit check-in task set; and
the fitness intervention sub-plan comprises a part or all of the first exercise plan.

12. The method according to claim 10 or 11, wherein the first intervention plan comprises the intervention plans of N cycles, and N is a positive integer greater than 1;

the wearable intervention sub-plan is a wearable intervention sub-plan of one or all cycles in the first intervention plan;
the check intervention sub-plan is a check intervention sub-plan of one or all cycles in the first intervention plan; and
the fitness intervention sub-plan is a fitness intervention sub-plan of one or all cycles in the first intervention plan.

13. The method according to any one of claims 1 to 12, wherein the first intervention plan comprises the intervention plans of N cycles, and N is a positive integer greater than 1; and the method further comprises:
obtaining, by the electronic device, actual execution data and/or a value of the health indicator in an execution process of a first cycle in the first intervention plan, wherein the actual execution data and/or the value of the health indicator are/is obtained through monitoring by the electronic device and/or another device in the health management system in which the electronic device is located.

14. The method according to claim 13, wherein the another device in the health management system in which the electronic device is located comprises the intelligent wearable device, and/or the health check device, and/or the intelligent fitness device.

15. The method according to claim 13 or 14, wherein a predicted value of the health indicator obtained after the first intervention plan is completed comprises a predicted value of the health indicator obtained after the first cycle in the first intervention plan is completed; and the method further comprises:
comparing, by the electronic device, the predicted value of the health indicator obtained after the first cycle in the first intervention plan is completed and an actual value of the health indicator obtained after an intervention plan of the first cycle is completed for a degree of consistency between the values, to obtain an intervention effect assessment result.

16. The method according to claim 15, wherein the method further comprises:
generating, by the electronic device, an assessment of one or more plans in the first intervention plan based on the intervention effect assessment result and with reference to the actual execution data and/or the value of the health indicator in the execution process of the first cycle, and using the assessment as an assessment result of the first intervention plan.

17. The method according to claim 16, wherein the method further comprises:
adjusting, by the electronic device, an intervention plan of a second cycle in the first intervention plan based on the assessment result of the first intervention plan, wherein the second cycle is a next cycle of the first cycle.

18. The method according to claim 17, wherein after the adjusting an intervention plan of a second cycle in the first intervention plan, the method further comprises:
predicting, by the electronic device, a predicted value of the health indicator obtained after a part and/or all of an adjusted first intervention plan is completed.

19. The method according to any one of claims 1 to 18, wherein the method further comprises:

estimating, by the electronic device, the age of the first user based on the user data, and using the age as an estimated user age of the first user, wherein the user data comprises the user behavior data and/or the user health data; and
predicting, by the electronic device, a predicted value of an estimated user age of the first user obtained after the part and/or all of the first intervention plan is completed.

20. The method according to claim 19, wherein the method further comprises:
displaying, by the electronic device, the estimated user age and the predicted value of the estimated user age.

21. An intervention plan assessment method, wherein the method comprises:

obtaining, by an electronic device, a predicted value of a health indicator obtained after a part and/or all of a first intervention plan is completed;
obtaining, by the electronic device, actual execution data and/or a value of the health indicator in an execution process of the first intervention plan; and
comparing, by the electronic device, the predicted value of the health indicator obtained after the part and/or all of the first intervention plan is completed and an actual value of the health indicator obtained after the part and/or all of the first intervention plan is completed for a degree of consistency between the values, to obtain an intervention effect assessment result.

22. The method according to claim 21, wherein the first intervention plan comprises intervention plans of N cycles, N is a positive integer greater than 1, and the predicted value of the health indicator obtained after the part and/or all of the first intervention plan is completed comprises a predicted value of the health indicator obtained after a first cycle in the first intervention plan is completed;
the obtaining, by the electronic device, actual execution data and/or a value of the health indicator in an execution process of the first intervention plan specifically comprises:

obtaining, by the electronic device, actual execution data and/or a value of the health indicator in an execution process of the first cycle in the first intervention plan, wherein the actual execution data and/or the value of the health indicator are/is obtained through monitoring by the electronic device and/or another device in a health management system in which the electronic device is located; and
the comparing, by the electronic device, the predicted value of the health indicator obtained after the part and/or all of the first intervention plan is completed and an actual value of the health indicator obtained after the part and/or all of the first intervention plan is completed for a degree of consistency between the values, to obtain an intervention effect assessment result specifically comprises:
comparing, by the electronic device, the predicted value of the health indicator obtained after the first cycle in the first intervention plan is completed and an actual value of the health indicator obtained after an intervention plan of the first cycle is completed for a degree of consistency between the values, to obtain the intervention effect assessment result.

23. The method according to claim 22, wherein the another device in the health management system in which the electronic device is located comprises an intelligent wearable device, and/or a health check device, and/or an intelligent fitness device.

**24.** The method according to claim 22 or 23, wherein the method further comprises:
generating, by the electronic device, an assessment of one or more plans in the first intervention plan based on the intervention effect assessment result and with reference to the actual execution data and/or the value of the health indicator in the execution process of the first cycle, and using the assessment as an assessment result of the first intervention plan.

**25.** The method according to claim 24, wherein the method further comprises:
adjusting, by the electronic device, an intervention plan of a second cycle in the first intervention plan based on the assessment result of the first intervention plan, wherein the second cycle is a next cycle of the first cycle.

**26.** The method according to any one of claims 21 to 25, wherein the method further comprises:

obtaining, by the electronic device, the first intervention plan generated based on user data of a first user; and
the obtaining, by an electronic device, a predicted value of a health indicator obtained after a part and/or all of a first intervention plan is completed specifically comprises:
predicting, by the electronic device, the predicted value of the health indicator obtained after the part and/or all of the first intervention plan is completed.

**27.** A user age estimation method, comprising:

obtaining, by an electronic device, user data of a first user, wherein the user data comprises user behavior data and/or user health data; and
estimating, by the electronic device, an age of the first user based on the user data of the first user, and using the age as an estimated user age of the first user.

**28.** The method according to claim 27, wherein the estimating, by the electronic device, an age of the first user based on the user data of the first user, and using the age as an estimated user age of the first user specifically comprises:

recognizing, by the electronic device, a user health risk factor based on the user data; and
estimating, by the electronic device, the age of the first user based on the user data and the user health risk factor, and using the age as the estimated user age of the first user.

**29.** The method according to claim 28, wherein the method further comprises:

generating, by the electronic device, a first intervention plan for the user health risk factor; and
predicting, by the electronic device, a predicted value of an estimated user age of the first user obtained after a part and/or all of the first intervention plan is completed.

**30.** The method according to claim 28 or 29, wherein the estimating, by the electronic device, the age of the first user based on the user data and the user health risk factor, and using the age as the estimated user age of the first user specifically comprises:

determining a baseline mortality rate for each cause-of-death disease of a first group corresponding to user basic information in the user data in a risk-free exposure case, wherein the risk-free exposure case is a hypothetical case in which everyone in the group has no health risk factor;
determining group life expectancy of the first group;
determining user life expectancy based on the user health risk factor and the baseline mortality rate for each cause-of-death disease of the first group in the risk-free exposure case; and
determining the estimated user age of the first user based on the user life expectancy, the group life expectancy of the first group, and an actual age of the first user.

**31.** An electronic device, wherein the electronic device comprises one or more processors and a memory; and
the memory is coupled to the one or more processors, the memory is configured to store computer program code, the computer program code comprises computer instructions, and the one or more processors invoke the computer instructions, so that the electronic device performs the method according to any one of claims 1 to 30.

**32.** A chip system, wherein the chip system is used in an electronic device, the chip system comprises one or more processors, and the processor is configured to invoke computer instructions, so that the electronic device performs the method according to any one of claims 1 to 30.

33. A computer-readable storage medium, comprising instructions, wherein when the instructions are run on an electronic device, the electronic device is enabled to perform the method according to any one of claims 1 to 30.

34. A user health management system, wherein the health management system comprises an electronic device and at least one of an intelligent wearable device, a health check device, or an intelligent fitness device, and the electronic device is configured to perform the method according to any one of claims 1 to 30.

Risk model
establishment

Model
inference

FIG. 1

```
┌──────────────┐      ┌──────────────────────────┐      ┌──────────────────────────┐      ┌──────────────────────────┐
│ Input basic  │      │   Health assessment      │      │   Health intervention    │      │ Execution monitoring     │
│ information, │      │                          │      │                          │      │      and feedback        │
│ sign data,   │ ───► │ ┌──────────────────────┐ │ ───► │ ┌──────────────────────┐ │ ───► │ ┌──────────────────────┐ │
│ and          │      │ │  Chronic disease     │ │      │ │     Exercise         │ │      │ │   Exercise plan      │ │
│ questionnaire│      │ │  risk assessment     │ │      │ │   intervention       │ │      │ │  execution status    │ │
│ data of a    │      │ └──────────────────────┘ │      │ │     solution         │ │      │ └──────────────────────┘ │
│ user         │      │                          │      │ └──────────────────────┘ │      │                          │
└──────────────┘      │ ┌──────────────────────┐ │      │                          │      │ ┌──────────────────────┐ │
                      │ │   Health risk        │ │      │ ┌──────────────────────┐ │      │ │ Daily dietary intake │ │
                      │ │   assessment         │ │      │ │  Dietary nutrition   │ │      │ └──────────────────────┘ │
                      │ └──────────────────────┘ │      │ │ intervention solution│ │      │                          │
                      │                          │      │ └──────────────────────┘ │      │ ┌──────────────────────┐ │
                      │ ┌──────────────────────┐ │      │                          │      │ │ Tracking and detection│ │
                      │ │   Risk factor        │ │      │ ┌──────────────────────┐ │      │ │ of sleep, stress, and │ │
                      │ │   recognition        │ │      │ │  Other intervention  │ │      │ │ sign indicators (body │ │
                      │ └──────────────────────┘ │      │ │ solutions (psychology,│ │      │ │ composition, blood    │ │
                      └──────────────────────────┘      │ │  sleep, smoking      │ │      │ │ pressure, blood       │ │
                                                        │ │ cessation, alcohol   │ │      │ │ glucose, and the like)│ │
                                                        │ │ restriction, and the │ │      │ └──────────────────────┘ │
                                                        │ │      like)           │ │      └──────────────────────────┘
                                                        │ └──────────────────────┘ │
                                                        └──────────────────────────┘
```

FIG. 2

Cloud 305

Electronic device 301

Intelligent wearable device 302

Health check device 303

Intelligent fitness device 304

FIG. 3

| Intelligent wearable device | Health check device | Intelligent fitness device | Electronic device |
|---|---|---|---|

(1)
Assessment

S401: Collect user data

S402: Recognize a user health risk factor

(2)
Intervention plan generation

S403: Generate personalized intervention plans of a plurality of cycles for the user health risk factor

(3)
Benefit prediction

S404: Predict, based on a benefit prediction model, a change trend of a health indicator obtained after an intervention plan is completed, and display the change trend to a user

(4)
Intervention execution

S405: Deliver the intervention plan

TO FIG. 4B     TO FIG. 4B     TO FIG. 4B

FIG. 4A

CONT.
FROM
FIG. 4A

CONT.
FROM
FIG. 4A

CONT.
FROM
FIG. 4A

(5)
Data
monitoring

S406: Monitor
actual execution
data of the
intervention plan
and health data

S407:
Measure the
health data

S408: Monitor the
actual execution
data of the
intervention plan

S409: Monitor the
actual execution
data of the
intervention plan
and the health data

S410: Send the
actual execution data
and the health data

(6)
Effect
assessment
and
intervention
plan
adjustment

S411: Run an effect assessment
model to assess an effect of an
actual execution status of an
intervention plan of a current
cycle, and provide a
recommendation

S412: Adjust an intervention
plan of a next cycle based on
an assessment result

FIG. 4B

**Basic user information 510**

User data:

| Date of birth | Gender | Height |

Collection manner:

Manually entered into a mobile terminal device by a user

**User behavior data 520**

User data:

| Exercise 521 | Stress 522 | Sleep 523 | Diet 524 | Drinking 525 | Smoking 526 |

Collection manner:

Collected by the mobile terminal device or an intelligent wearable device

Photo recognition

Recorded by an intelligent fitness device

Manually entered into the mobile terminal device by the user

**User health data 530**

User data:

| Body weight | Body composition | Blood pressure | Blood glucose | Blood lipid |

Collection manner:

Recorded by a health check device

| Intelligent body fat scale | Intelligent sphygmomanometer | Blood glucose meter | Blood lipid monitor |

Sent to the mobile terminal device by the health check device connected to a network, or manually entered into the mobile terminal device by the user

FIG. 5

```
┌ ─ ─ ─ ─ ─ ─ ─ ─ ─ ─ ─ ─ ─ ─ ┐              ┌──────────────────────────────┐
│  ┌────────────────────────┐  │              │ Basic user information, user │
│  │     GBD database       │  │              │   behavior data, and user    │
│  └────────────────────────┘  │              │         health data          │
│              │               │              └──────────────────────────────┘
│              ▼               │                            │
│  ┌────────────────────────┐  │              ┌─────────────▼────────────────┐
│  │  Risk factors related  │  │─────────────▶│  Set of risk factors related │
│  │   to all causes of     │  │              │        to a lifestyle        │
│  │        death           │  │              └──────────────────────────────┘
│  └────────────────────────┘  │                            │
└ ─ ─ ─ ─ ─ ─ ─ ─ ─ ─ ─ ─ ─ ─ ┘                            ▼
┌────────────────────────┐                    ┌──────────────────────────────┐
│ Guidelines, consensus, │───────────────────▶│ Health risk factor item and  │
│  and cohort research   │                    │     threshold database       │
│      literature        │                    └──────────────────────────────┘
└────────────────────────┘                                  │
                                                            ▼
                                              ┌──────────────────────────────┐
                                              │    User health risk factor   │
                                              └──────────────────────────────┘
```

FIG. 6

| Data preparation | | Electronic device |

SS701: Obtain a complete set of risk factors that affect health

SS702: Select health risk factors that are related to a lifestyle and a daily behavior and that are daily controllable by a user

SS703: Establish a subgroup health risk factor item and threshold database

SS704: Obtain a health risk factor item and threshold database of a first group corresponding to basic user information

SS705: Determine a user health risk factor based on user behavior data, user health data, and the health risk factor item and threshold database of the first group

FIG. 7

FIG. 8

EP 4 369 357 A1

| User health risk factor | Personalized information | | Solution and plan |
|---|---|---|---|

**User health risk factor**

- Insufficient physical activity
- Unbalanced nutrition or unhealthy eating habit
- Sleep deprivation or sleep disorders
- Excessive stress
- Smoking
- Excessive drinking

**Personalized information**

- Age
- Gender
- Exercise preference
- Physical fitness assessment result
- Diet habit
- Disease history

**Lifestyle intervention recommendation rule base**

**Solution and plan**

- Exercise plan and objective → Weekly exercise plan (intensity, frequency, time, type, and taboo)
- Diet plan and objective → Daily diet plan (proportion and total amount of nutrition intake, and type and weight of food)
- Daily early bed task check-in, and sleep improvement plan (meditation, deep sleep exercise, and soothing relaxation before bed)
- Daily breathing training task check-in, and relaxation plan (sleep and mindfulness)
- Smoking cessation plan (daily reduction, immediate withdrawal, or daily delay based on smoking severity)
- Alcohol restriction plan (daily reduction based on excessive drinking severity)

FIG. 9

FIG. 10

Basic group model training         Individual model training          Individual model inference

Cloud                              Electronic device                  Electronic device

Group training data                Individual training data

Execution status of an             Execution status of a              To-be-executed
intervention plan of a group       historical intervention plan of    intervention plan of
user                               the user                           the user

Value of a health indicator        Value of the health indicator      Current value of the
before the intervention plan is    before the user executes the       health indicator
executed                           intervention plan

Value of the health indicator      Value of the health indicator
after the intervention plan is     after the user executes the
executed                           intervention plan

                                                                      Individual health benefit
                                                                      prediction model

Machine learning algorithm         Basic group model

                                                                      Prediction
                                                                      result

Model verification                 Model parameter fine-tuning        Value of the health indicator after
                                                                      intervention is executed

FIG. 11

EP 4 369 357 A1

```
┌─────────────────────────────────────────────────────────────────────────┐
│                 ┌──────────────────────┐      ┌──────────────────┐        │
│                 │ Data after weekly    │      │                  │        │
│                 │ intervention plan    │      │   Neural network │        │
│                 │ execution is completed│─────▶│                  │        │
│                 │ and health indicator │      │                  │        │
│                 │ before an intervention│      └──────────────────┘        │
│                 │ plan is executed     │                                   │
│   ┌──────────┐  └──────────────────────┘      ┌──────────────────┐        │
│   │ Training │                                 │ Predicted health │        │
│   │ data set │                                 │ indicator after  │        │
│   └──────────┘                                 │ the intervention │        │
│                 ┌──────────────────────┐      ┌──────────────────┐        │
│                 │ Actual health        │      │ Optimize an      │        │
│                 │ indicator after the  │─────▶│ objective        │        │
│                 │ intervention plan is │      │ function         │        │
│                 │ executed             │      └──────────────────┘        │
│                 └──────────────────────┘              Model training       │
└─────────────────────────────────────────────────────────────────────────┘
```

FIG. 12

| Cloud | | Electronic device |
|---|---|---|

SS1301: Obtain basic group models through training by using a machine learning algorithm and training data of various groups

SS1302: Obtain a basic group model of a first group matching basic user information

SS1303: Train the basic group model of the first group based on individual training data of a user, to obtain a first individual health benefit prediction model

SS1304: Obtain, through prediction based on a part and/or all of a first intervention plan of the user, a current value of a health indicator of the user, and the first individual health benefit prediction model, a predicted value of the health indicator obtained after the part and/or all of the first intervention plan is executed

FIG. 13

| Intelligent wearable device | Health check device | Intelligent fitness device | Electronic device |
|---|---|---|---|

Part or all of a first exercise plan

Part or all of health indicator check tasks in a first health habit check-in task set

Part or all of the first exercise plan, a first diet plan, and/or the first health habit check-in task set

FIG. 14

| Execute a plan and predict a benefit | Actual execution monitoring |
|---|---|
| Predicted value of a post-intervention health indicator | Value of the health indicator after actual completion |
| Intervention plan | Actual intervention plan completion status |

| Consistency degree comparison | Exercise and diet joint assessment rule base |
|---|---|

| Intervention effect assessment | Joint assessment |
|---|---|

FIG. 15

An electronic device compares a predicted value of a health indicator obtained after an intervention plan of a cycle in a first intervention plan is completed and a value of the health indicator obtained after the intervention plan of the cycle is actually completed for a degree of consistency between the values, to obtain an intervention effect assessment level ⟍ SS1601

The electronic device generates an assessment and a recommendation on one or more plans in the first intervention plan based on the intervention effect assessment level, the intervention plan of the cycle, and behavior data obtained after the intervention plan of the cycle is actually completed ⟍ SS1602

FIG. 16

FIG. 17

S1801

Determine an average group mortality rate for each cause-of-death disease of a first group to which a user belongs

S1802

Determine a baseline mortality rate for each cause of death of the first group in a risk-free exposure case

S1803

Determine group life expectancy of the first group

S1804

Determine user life expectancy based on a user health risk factor and the baseline mortality rate of the first group

S1805

Determine a user health age based on the group life expectancy of the first group and the user life expectancy

FIG. 18

Basic group model training      Individual model training      Individual model inference

**Cloud**

Group training data

| Execution status of an intervention plan of a group user |

| Value of a health indicator and user health age before the intervention plan is executed |

| Value of the health indicator and user health age after the intervention plan is executed |

↓

Machine learning algorithm

↓

Model verification

**Electronic device**

Individual training data

| Execution status of a historical intervention plan of the user |

| Value of the health indicator and user health age before the user executes the intervention plan |

| Value of the health indicator and user health age after the user executes the intervention plan |

↓

Basic group model

↓

Model parameter fine-tuning

**Electronic device**

| To-be-executed intervention plan of the user |

| Current value of the health indicator and current user health age |

↓

Individual health benefit prediction model

Prediction result

↓

Value of the health indicator and user health age after the intervention plan is executed

FIG. 19

EP 4 369 357 A1

FIG. 20

| Execute a plan and predict a benefit | Actual execution monitoring |
|---|---|
| Predicted value of a post-intervention health indicator | Value of the health indicator after actual completion |
| Predicted post-intervention user health age | User health age after actual completion |
| Intervention plan | Actual intervention plan completion status |

| Consistency degree comparison | Exercise and diet joint assessment rule base |
|---|---|

| Intervention effect assessment | Joint assessment |
|---|---|

FIG. 21

An electronic device compares a predicted value of a health indicator obtained after an intervention plan of a cycle in a first intervention plan is completed and an actual value of the health indicator obtained after the intervention plan of the cycle is completed for a degree of consistency between the values, and compares a predicted value of a user health age obtained after the intervention plan of the cycle is completed and an actual value of the user health age obtained after the intervention plan of the cycle is completed for a degree of consistency between the values, to obtain an intervention effect assessment level ⟋ SS2201

The electronic device generates an assessment and a recommendation on one or more plans in the first intervention plan based on the intervention effect assessment level, the intervention plan of the cycle, and behavior data obtained after the intervention plan of the cycle is actually completed ⟋ SS2202

FIG. 22

Data collection module

| Basic Information | Sign data | | | Behavior data | | |
|---|---|---|---|---|---|---|
| Age | Blood pressure | Blood glucose | Blood lipid | Exercise | Diet | Sleep habit |
| Gender | Body weight and body fat | Stress | Sleep stages | Drinking | Smoking | Health questionnaire |

Health risk factor recognition and health age assessment module

| Health age prediction | Health risk factor and risk degree ranking |
|---|---|

Personalized lifestyle intervention solution generation module

| Exercise intervention solution and plan | Diet intervention solution and plan | Sleep intervention solution |
|---|---|---|

| Psychological and stress intervention solution | Intervention solutions for bad lifestyles such as smoking and excessive drinking |
|---|---|

Health benefit prediction model

Plan completion health benefit prediction

Health age change trend prediction

Intervention solution execution monitoring module

Exercise/diet behavior monitoring

Health indicator check

Behavior-based compliance management module

Point reward

Actual execution effect joint assessment module

| Effect assessment and comprehensive assessment | Dynamic adjustment of intervention plan |
|---|---|

FIG. 23

Electronic device 100

Antenna 1

Antenna 2

| Mobile communication module 2G/3G/4G/5G [150] | Wireless communication module BT/WLAN/GNSS/NFC/IR/FM [160] |
|---|---|

Speaker [170A]

Receiver [170B]

Microphone [170C]

Headset jack [170D]

Audio module [170]

Displays 1 to N [194]

Cameras 1 to N [193]

Indicator [192]

Motor [191]

Button [190]

Internal memory [121]

SIM card interfaces 1 to N [195]

External memory interface [120]

USB interface [130]

Charging input

Charging management module [140]

Processor [110]

Power management module [141]

Battery [142]

Sensor module [180]

Pressure sensor [180A]

Gyroscope sensor [180B]

Barometric pressure sensor [180C]

Magnetic sensor [180D]

Acceleration sensor [180E]

Distance sensor [180F]

Optical proximity sensor [180G]

Fingerprint sensor [180H]

Temperature sensor [180J]

Touch sensor [180K]

Ambient light sensor [180L]

Bone conduction sensor [180M]

FIG. 24

## INTERNATIONAL SEARCH REPORT

| International application No. |
| --- |
| **PCT/CN2022/113387** |

| A. | CLASSIFICATION OF SUBJECT MATTER |
| --- | --- |
| | G16H 50/30(2018.01)i; G16H 50/30(2018.01)i |

According to International Patent Classification (IPC) or to both national classification and IPC

| B. | FIELDS SEARCHED |
| --- | --- |

Minimum documentation searched (classification system followed by classification symbols)

G16H

Documentation searched other than minimum documentation to the extent that such documents are included in the fields searched

Electronic data base consulted during the international search (name of data base and, where practicable, search terms used)

CNPAT, CNKI, WPI, EPODOC: 干预, 治疗, 计划, 方案, 预测, 预计, 估计, 估测, 健康, 对比, 比较, 评估, 评价, 风险, 危险, 行为, 年龄, 年纪, 寿命, intervention, treatment, plan, program, predict, estimate, health, compare, evaluate, assessment, risk, hazard, danger, behavior, action, age, lifetime

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 109727676 A (HEFEI INSTITUTES OF PHYSICAL SCIENCE, CHINESE ACADEMY OF SCIENCES) 07 May 2019 (2019-05-07) description, paragraphs [0004]-[0041] | 1-18, 21-26, 31-34 |
| Y | CN 109727676 A (HEFEI INSTITUTES OF PHYSICAL SCIENCE, CHINESE ACADEMY OF SCIENCES) 07 May 2019 (2019-05-07) description, paragraphs [0004]-[0041] | 19-20 |
| X | CN 109785964 A (PING AN TECHNOLOGY (SHENZHEN) CO., LTD.) 21 May 2019 (2019-05-21) description, paragraphs [0006]-[0044] and [0092]-[0126] | 27-34 |
| Y | CN 109785964 A (PING AN TECHNOLOGY (SHENZHEN) CO., LTD.) 21 May 2019 (2019-05-21) description, paragraphs [0006]-[0044] and [0092]-[0126] | 19-20 |
| X | JP 6901169 B1 (NISSHIN BUSINESS DEV CO., LTD.) 14 July 2021 (2021-07-14) description, paragraphs [0009]-[0024] | 27-34 |
| Y | JP 6901169 B1 (NISSHIN BUSINESS DEV CO., LTD.) 14 July 2021 (2021-07-14) description, paragraphs [0009]-[0024] | 19-20 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 November 2022** | **24 November 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/113387** |

### C. DOCUMENTS CONSIDERED TO BE RELEVANT

| Category* | Citation of document, with indication, where appropriate, of the relevant passages | Relevant to claim No. |
| --- | --- | --- |
| A | US 2018211727 A1 (BASEHEALTH, INC.) 26 July 2018 (2018-07-26)<br>entire document | 1-26, 31-34 |
| A | CN 102077228 A (MICROSOFT CORP.) 25 May 2011 (2011-05-25)<br>entire document | 27-34 |

Form PCT/ISA/210 (second sheet) (January 2015)

**INTERNATIONAL SEARCH REPORT**

International application No.

**PCT/CN2022/113387**

| Box No. III | Observations where unity of invention is lacking (Continuation of item 3 of first sheet) |

This International Searching Authority found multiple inventions in this international application, as follows:

[1] A first group of claims (1, 21, and 31-34) relates to a method in which an electronic device acquires a first intervention plan, and obtains an intervention effect evaluation result on the basis of the first intervention plan.

[2] A second group of claims (27 and 31-34) relates to a method in which an electronic device acquires user data of a first user, and evaluates the age of the first user according to the user data of the first user.

[3] When referring to claims 1-26 and claims 27-30, claims 31-34 belong to the first group of claims and the second group of claims, respectively.

[4] The same or corresponding technical feature between the described two groups of claims only lies in: executing an operation by an electronic device. However, the above feature is a conventional means in the relevant field and does not make a contribution over the prior art.

[5] Therefore, the described two groups of claims are not linked with each other by a same or corresponding special technical feature, and define two different inventions, and these inventions are not linked with each other by a single general inventive concept. Therefore, the present application lacks unity of invention as defined in PCT Rules 13.1 and 13.2.

1. [✓] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[✓] No protest accompanied the payment of additional search fees.

Form PCT/ISA/210 (continuation of first sheet) (January 2015)

**INTERNATIONAL SEARCH REPORT**
Information on patent family members

International application No.

**PCT/CN2022/113387**

| Patent document cited in search report | | | Publication date (day/month/year) | Patent family member(s) | | | Publication date (day/month/year) |
|---|---|---|---|---|---|---|---|
| CN | 109727676 | A | 07 May 2019 | None | | | |
| CN | 109785964 | A | 21 May 2019 | None | | | |
| JP | 6901169 | B1 | 14 July 2021 | None | | | |
| US | 2018211727 | A1 | 26 July 2018 | None | | | |
| CN | 102077228 | A | 25 May 2011 | US | 2009327076 | A1 | 31 December 2009 |
| | | | | KR | 20110050406 | A | 13 May 2011 |
| | | | | WO | 2009158090 | A2 | 30 December 2009 |
| | | | | EP | 2291807 | A2 | 09 March 2011 |
| | | | | JP | 2011527040 | A | 20 October 2011 |

Form PCT/ISA/210 (patent family annex) (January 2015)

**REFERENCES CITED IN THE DESCRIPTION**

**Patent documents cited in the description**

- CN 202111000243